# EUROPÄISCHE PATENTSCHRIFT

(11) **EP 0 854 938 B1**
(45) Veröffentlichungstag und Bekanntmachung des Hinweises auf die Patenterteilung: **07.12.2005**
(21) Anmeldenummer: 96934572.7
(22) Anmeldetag: 08.10.1996
(51) Int. Cl.: C12Q 1/68, C07H 21/00

(54) **VERZWEIGTE SONDE UND VERFAHREN ZUM NACHWEIS EINES ANALYTEN**
BRANCHED PROBE AND METHOD OF DETECTING A SUBSTANCE TO BE ANALYZED
SONDE RAMIFIEE ET PROCEDE DE MISE EN EVIDENCE D'UN ANALYTE

(30) Priorität: 12.10.1995 DE 19537952
(43) Veröffentlichungstag der Anmeldung: 29.07.1998
(73) Patentinhaber: Roche Diagnostics GmbH, 68305 Mannheim (DE)
(72) Erfinder: WEINDEL, Kurt, 82407 Wielenbach (DE); SEIDEL, Christoph, 82362 Weilheim (DE); LASSONCZYK, Gerhard, 82380 Peissenberg (DE)
(86) Internationale Anmeldenummer: PCT/EP1996/004358
(87) Internationale Veröffentlichungsnummer: WO 1997/013874

(56) Entgegenhaltungen:
- EP-A- 0 137 515
- EP-A- 0 324 474
- EP-A- 0 523 557
- WO-A-90/13667
- WO-A-95/01365

## Beschreibung

Gegenstand der vorliegenden Erfindung ist ein Verfahren zum Nachweis eines Analyten in einer Probe mit Hilfe einer nukleobasenhaltigen Sonde.

Der Nachweis von niedrigkonzentnenen Analyten in Proben ist in jüngerer Zeit in den Mittelpunkt des Interesses der klinischen Analytik geruckt. Zahlreiche Entwicklungen haben es ermöglicht, daß die Nachweisgrenze fur Analyten verglichen mit früheren Tests erheblich gesenkt werden konnte Dies hat sich insbesondere auf dem Gebiet des Nachweises von Nukleinsäuren bemerkbar gemacht Viele der derzeit verwendeten Nachweissysteme sind jedoch entweder in der Verfahrensführung oder in der Herstellung dafür geeigneter Reagenzien sehr aufwendig und oft auch mit Nachteilen behaftet.

In US-A-4,683,202 ist ein Verfahren zum Nachweis von Nukleinsäuren beschrieben, welches auf der Amplifikation von Segmenten einer ursprünglichen Nukleinsäure beruht. Dieses Verfahren ist als Polymerase-Kettenreaktion bekannt geworden. Ein Nachteil dieses Verfahrens ist jedoch die schwierige Quantifizierbarkeit des Analyseergebnisses.

Bessere Ergebnisse im Hinblick auf die Quantifizierung der Analyse wurden durch Benutzung von Nukleinsäuresonden erreicht, bei denen eine im Regelfall stöchiometrische Hybridisierung erfolgt Diese Verfahren haben jedoch wiederum den Nachteil, daß sie wenig sensitiv sind.

In einer Weiterentwicklung der üblichen Hybridisierungstests wurde daher vorgeschlagen, mit jeder Sonde eine Vielzahl von identischen Nukleotidsequenzen zu verbinden. Das Hybrid aus Analytnukteinsäure und Sonde wurde durch Hybridisierung einer Vielzahl von zu diesen identischen Nukleotidsequenzen komplementären Sekundärsonden nachgewiesen Eine Vielzahl von Dokumenten zum Stand der Technik beschäftigt sich mit der günstigsten Anordnung der Vielzahl von identischen Nukleotidsequenzen in der Sonde bzw in einem Set aus miteinander hybridisierungs- und somit aggregationsfähigen Sonden (u.a. US 5,424,188; EPS 0 248 896 B1; US 5,424,413; US 5,437,977). Beispielsweise ist in US-A-5,424,188 beschrieben, daß die identischen Nukleotidsequenzen linear aneinander gehängt werden können. Die entstandene Kette von identischen Nukleotidsequenzen kann auch zu einem Zyklus geschlossen sein. In US-A-5,124,246 ist beschrieben, daß die identischen Nukleotidsequenzen über eine verzweigte Struktur miteinander verbunden sein können. Es wird hier zwischen gabelartigen Verzweigungen und kammartigen Verzweigungen unterschieden. Für die technische Ausführung der Verzweigung wurden mehrere Möglichkeiten beschrieben (Nucleic Acids Research 16/11, 4937 - 4956, Gene 61, 253 - 264, Nucleic Acids Research 17/17, 6959 - 6967, Clinical Chemistry 35/8, 1571 - 1575 (1989), Bioorganic and Medicinal Chemistry Letters 4/8, 1011 - 1018 (1994), Nucleic Acids Research Symposium 24, 197 - 200 (1991) und Clin. Chem.39/4, 725 - 726 (1993)). In WO 9S/01365 ist ebenfalls ein Verzweigungsverfahren beschrieben, bei dem die Seitenarme über eine Reaktion zwischen Phosphorothioaten und Haloazylgruppen an ein Sondenrückgrat gebunden werden. Verfahren, bei denen die Sonde eine Vielzahl von identischen Nukleotidsequenzen zur Hybridisierung mit Sekundärsonden aufweist, können oftmals zu einer Erhöhung der Eichkurvensensitivität führen. Die hierfür benötigten Sonden sind jedoch 1.) schwer herzustellen und 2.) findet keine stöchiometrische Absättigung der Nukleinsäuresequenzen an den Sonden durch die markierten Sonden statt. Die erreichte Testsensitivität bleibt demnach deutlich hinter der der PCR-Methode zurück (AIDS 7/suppl., 11 - 14 (1993); J. Med. Virol. 43, 262 - 268 (1994); J. Infect. Dis. 170, 1172 - 1179 (1994); AIDS Res. & Human Retrovir. 11/3, 353 - 361 (1995).

Allen diesen Konzepten gemeinsam ist die Aggregation mindestens zweier verschiedener Sorten von Sonden, von denen eine in einer Vielzahl vorhanden, ist nach dem Prinzip der Hybridisierung einzelsträngiger Überhänge zu einer nachweisbaren Amplifikationseinheit.

Gegenstand der Erfindung war es nun, den Stand der Technik zu verbessern und insbesondere eine Sonde und eine empfindliche und direkt-quantifizierende Nachweismethode bereitzustellen.

Gegenstand der Erfindung ist daher eine Sonde mit einem analytischen Teil einem Teil, der Nukleobasen an einem Backbone enthält und ein Verfahren zum Nachweis eines Analyten in einer Probe durch Kontaktieren der Probe mit der Sonde unter Bedingungen, bei denen der Analyt mittelbar oder unmittelbar an die nukleobasenhaltige Sonde bindet und Nachweis des Bindungsprodukts, wobei die Sonde markierungsvermittelnde, nicht-nukleosidische Gruppen aufweist.

Ein Analyt im Sinne des erfindungsgemäßen Verfahrens kann jeder Inhaltsstoff einer Probe sein. Insbesondere handelt es sich bei dem Analyten jedoch um ein immunologisch oder über Basenpaarung erkennbares Molekül. Immunologisch erkennbare Analyten sind z. B. Antikörper, Antigene oder Haptene. Über Basenpaarung erkennbare Analyten sind Nukleinsäuren, z. B. Ribonukleinsäuren und Deoxyribonukleinsäuren. Solche Analyten können mit dem vorliegenden Verfahren in praktisch allen Proben nachgewiesen werden. In vielen Fällen hat es sich jedoch als bevorzugt erwiesen, die Probe so vorzubehandeln, daß der Analyt in einer für den Nachweis besser geeigneten Form vorliegt. Hierzu gehört beispielsweise die Freisetzung des Analyten aus Kompartimenten, z. B. Zellen, in die der Analyt ursprünglich eingeschlossen war. Bei dem Analyten kann es sich jedoch auch um ein Kompartiment selbst handeln, wenn dieses an seiner Oberfläche erkennbare Bestandteile, z. B. Oberflächenantigene, aufweist. Außerdem kann der eigentliche Analyt auch vor dem erfindungsgemäßen Verfahren amplifiziert sein, z.B. mittels der PCR, bevorzugt aber nur bis zu einem Maß, bei dem die Amplifikation noch quantifizierbar ist. Als geeignete Flüssigkeiten, aus denen eine zum Nachweis geeignete Probe hergestellt werden kann, haben sich Blut, Urin, Sputum oder Abstriche erwiesen. Als ein weiterer möglicher Vorbereitungsschritt kann die Verflüssigung von zähen Proben oder die partielle Aufreinigung des Analyten, z. B. durch Immobilisierung an einer Festphase, angesehen werden. Der Analyt kann in einer Flüssigkeit vorliegen, z. B. in gelöster oder suspendierter Form. Der Analyt kann jedoch auch an eine Festphase gebunden sein. Als Festphase können beispielsweise Latexpartikel, Magnetpartikel oder Wände von Gefäßen eingesetzt werden.

Als Sonde im Sinne der vorliegenden Erfindung wird ein Molekül verstanden, welches einen ersten Teil, der Nukleobasen an einem Backbone enthält, und einen zweiten, analytspezifischen bzw. zum Analyten hin kontaktvermittelnden Teil aufweist. Nukleobasen sind z. B. die natürlich vorkommenden Basen A, G, C, T und U oder auch hiervon abgeleitete Basen. Als Backbone kommt beispielsweise das natürliche Zuckerphosphatgerüst in Frage. In jüngerer Zeit wurden auch Moleküle, die einen Polyamid-Backbone besitzen, beschrieben (z. B. WO 91/20702). Mit Hilfe des analytspezifischen Teils kann die Sonde den Analyten direkt oder indirekt binden. Für den Fall einer direkten Bindung hängt die Natur des analytspezifischen Teils von der Natur des Analyten ab. Handelt es sich um einen immunologisch aktiven Analyten, kann die Bindung über den immunologisch komplementären Partner zum Analyten geschehen. So kann z. B. für den Nachweis eines Antigens eine Sonde verwendet werden, welche einen Antikörper gegen dieses Antigen kovalent oder über nicht-kovalente Bindung an den nukleinsäureartigen Teil gebunden enthält. Ist der Analyt eine Nukleinsäure, so enthält die Sonde eine Nukleobasensequenz, die zu einem Teil der Basensequenz des Analyten komplementär ist. Eine indirekte Bindung der Sonde an den Analyten kann dadurch erreicht werden, daß der analytspezifische Teil gegen einen Teil einer Vermittlersonde gerichtet ist, welche an den Analyten binden kann. Die Vermittlersonde enthält daher bevorzugt einen analytspezifischen Teil und einen Teil, der zum analytspezifischen Teil der Sonde bindefähig ist. Für diesen Fall ist es bevorzugt, daß die Sonde und die Vermittlersonde über Basenpaarung aneinander binden. In diesem Fall wird die Vermittlersonde bevorzugt so gewählt, daß sie einen zu dem Analyten komplementären Teil und einen zu der Sonde komplementären Teil aufweist. Dieses Vorgehen hat den Vorteil, daß für den Nachweis verschiedener Analyte zwar eine der Anzahl der nachzuweisenden Analyten entsprechende Anzahl von relativ einfach aufgebauten Vermittlersonden eingesetzt werden muß, jedoch nur eine relativ komplexe Sonde.

Der Teil der Sonde, der Nukleobasen an einem Backbone enthält, ist so gestaltet, daß er nicht mit anderen, in der Reaktionsmischung oder Probenmischung vorkommenden Nukleinsäuren, Hybride bilden kann. Dieser Teil ist sterisch anspruchsvoll, insbesondere mehrfach in sich verzweigt. Diese Verzweigung ist bevorzugt chemisch kovalent, besonders bevorzugt über funktionelle Gruppen zwischen (Deoxy-)Riboseeinheiten, verzweigt. Bevorzugt handelt es sich außerdem um einen Teil, der eine Vielzahl identischer Nukleotidsequenzen enthält. Die Herstellung solcher verzweigter Moleküle ist in dem anfangs beschriebenen Stand der Technik ausführlich beschrieben. Im Folgenden sollen einige Konzepte zur Herstellung verzweigter Teile, die Nukleobasen an einem Backbone enthalten, dargelegt werden.

In einer ersten Möglichkeit werden Nukleinsäuren, die durch Einbau von N⁴-(N-(6-Tri-fluoroazethyl-amidocaproyl)-2-aminoethyl)-2'-deoxycytidin im Rahmen der Phosphoramiditsynthese hergestellte Nukleinsäuren mit Hilfe von p-phenylendiisothyocyanat (DITC) als homo-bi funktionellem Linker quervernetzt und eine geeignete doppelsträngige Fraktion aus dem Produktgemisch isoliert, z. B. stemförmige Multimere. Die Herstellung dieser Multimere ist in "Luminescence-Immunoassay and Molecular Applications" (1990, Editor: Knox Van Dyke, CRC Press, Boca Raton, USA) beschrieben. Eine weitere Möglichkeit der Verzweigung bieten sogenannte Verzweigungsmonomere, die insgesamt 3 Hydroxylgruppen pro Nukleotid aufweisen. Die zusätzliche, orthogonal zur 5', 3'-Achse stehende Hydroxylgruppe kann beispielsweise in Cytidin in Stellung N4 über eine Hexamethylengruppe gebunden sein. Während der Oligonukleotidsynthese nach dem Phosphoramiditverfahren wird bei der Umsetzung mit einem Nukleosidphosphoramidit nicht nur die 5'-Hydroxylgruppe reagieren, sondern auch die an der Base befindliche (ehemals mit derselben Schutzgruppe versehene) Hydroxylgruppe. Hierdurch verzweigt sich die im Aufbau befindliche Oligonukleotidkette jedes Mal, wenn ein Verzweigungsmonomer eingesetzt wird. Durch Variation der Schutzgruppen ist es möglich, zunächst einen linearen DNA-Strang zu synthetisieren, der aber überall da, wo solche Verzweigungsmonomere inkorporiert wurden, Verzweigungspunkte besitzt. Anschließende selektive Abspaltung der Schutzgruppe der zweiten Hydroxylgruppe ermöglicht die separate Synthese von Seitensträngen. Diese Sekundärsequenzen gehen wie Zähne eines Kammes von der Primärsequenz ab (Kammstrukturen). Diese Verfahren sind beispielsweise in Nucleic Acids Research 17/17, 6959-6967 (1989) und Nucleic Acids Research Symposium 24, 197 - 200 (1991) beschrieben.

Ein Kern der Erfindung ist, daß die Sonde innerhalb des Teils, der Nukleobasen an einem Backbone enthält, markierungsvermittelnde, nicht-nukleosidische Gruppen aufweist. Vom Stand der Technik unterscheidet sich daher die Erfindung dadurch, daß die bisher als Markierungsvermittler eingesetzten langen identischen Nukleotidsequenzen durch niedermolekulare relativ kleine, in vergleichsweise dichten Abständen voneinander inkorporierte, (bevorzugt vom Backbone ausgehend flexibel gespacerte) nicht-nukleosidische Gruppen ersetzt werden. Diese markierungsvermittelnden Gruppen sind sterisch wenig anspruchsvoll. Bevorzugte markierungsvermittelnde Gruppen sind relativ kleine immunologisch erkennbare Reste, wie z. B. Haptene. Besonders bevorzugt im Sinne der Erfindung sind gut solvatisierbare Haptene, die eine starke antigene Determinante darstellen. Kandidaten sind z. B. Digoxigenin (US-A-5,344,757), Nitrophenole (wie z. B. Dinitrophenol oder Nitrojodphenol), spezielle Zucker, wie z. B. Lactosamin, oder geeignete Arzneimittel. Beispielhaft aufgeführt werden können die 4-Hydroxy-3-nitro-phenacetyl-Gruppe (NP), z. B. als Carboxamid via EDC an einen Aminospacer wie Aminocapronsäure gekoppelt, und weiter aktiviert durch Veresterung mit NHS zum (N-Hydroxysuccinimidyl)-ε-aminocaproyl-NP-carboxamid (damit können dann z. B. Aminoalkyl-derivatisierte Phosphonat-Brücken, Nukleinbasen oder Zucker angegriffen werden), die 4-Hydroxy-3-iodo-5-nitro-phenacetyl-Gruppe (NIP, z. B. als Carboxamid via EDC an einen Aminospacer wie Aminocapronsäure gekoppelt, und weiter aktiviert durch Veresterung mit NHS zum (N-Hydroxysuccinimidyl)-ε-amino-caproyl-NIP-carboxamid (damit können dann Aminoalkyl-derivatisierte Phosphonat-Brücken, Nukleinbasen oder Zucker angegriffen werden; spezifische <NIP>-Antikörper sind beschrieben) und 1-Dimethoxytrityl-3-O-((N-(2,4-dinitrophenyl)-3-aminopropyl)-triethylenglycol)-glyceryl-2-O-(cyanoethyl)-(N,N-diisopropyl)-phosphoramidit (DNP-TEG) (DNP-TEG kann mittels des Phosphitverfahrens im Laufe der DNA-Synthese direkt eingebaut werden; spezifische <DNP>-Antikörper sind beschrieben).

Die markierungsvermittelnden Gruppen können in den Teil, der Nukleobasen an einem Backbone enthält, im Anschluß an dessen Synthese eingebracht werden. Dies kann beispielsweise geschehen durch Umsetzung des nukleinsäurespezifischen Teiles mit Verbindungen, die zur kovalenten Bindung von Resten mit Nukleinsäuren in der Lage sind. Hierbei handelt es sich insbesondere um Reagenzien, die mit funktionellen Resten der Nukleinsäure, z. B. von exozyklischen Aminogruppen der Basen geeignet sind. Ein geeignetes Verfahren ist beispielsweise in EP-A-0 173 251 beschrieben. Ferner ist es möglich, die markierungsvermittelnde Gruppe durch Photoverknüpfung der Nukleinsäure mit einem photoaktivierbarem Reagenz herzustellen. Ein solches Verfahren ist beispielhaft beschrieben in US-A-5,344,757. Dort ist die Markierung mit einem Digoxigeninrest beschrieben.

Es ist jedoch auch möglich, die markierungsvermittelnde Gruppe in bereits an die während der Synthese des Teils, der Nukleobasen an einem Backbone enthält, verwendeten Mononukleotidbausteine gebundenen Form einzusetzen. Beispielsweise können in der Phosphoramiditsynthese Phosphoramidite eingesetzt werden, bei denen an exozyklische Aminogruppen einer Base, oder an den entstehenden Internucleosid-Phophodiestern, markierungsvermittelnde Gruppen oder geschützte Vorläuferreste gebunden sind.

Bei den erfindungsgemäßen Sonden handelt es sich bevorzugt um einzelsträngige, chemisch synthetisierte Verbindungen.

Ein weiteres mögliches Verfahren zur Herstellung basiert auf dem Syntheseweg, wie er in WO 95/01365 beschrieben ist. Grundlage ist die sehr effiziente Kopplung von Thiophosphat oder Dithiophosphatgruppen und Haloazyl- oder Haloalkylgruppen. Bevorzugt werden Thiophosphat- und Betabromazetamidofunktionen eingesetzt, die unter Bindung von Thiophosphorylazetamidobrücken koppeln. Verzweigte Multimere, die als Teil, der Nukleobasen an einem Backbone enthält, eingesetzt werden können, können in folgender Weise hergestellt werden. In einem ersten Schritt wird ein am 5'-Ende Amino-oder Aminoalkyl-derivatisiertes erstes Oligonukleotid mit N-hydroxysuccinimedyl-beta-Brom-azetat unter Bildung einer terminalen Beta-Brom-Azetamido-Funktion umgesetzt. In einem zweiten Schritt wird ein zweites Oligonukleotid am 3'-Ende als 3'-O-phosphoryl-(2-aminomethyl-)ethyl-thiophosphatester funktionalisiert. Die terminale Thiophosphatgruppe des zweiten Oligonukleotids reagiert dann unter nukleophilem Angriff an das bromsubstituierte Beta-C-Atom am 5'-Ende des ersten Oligonukleotids unter Ligation beider Oligonukleotide. Anschließend wird die Aminomethylfunktion zu einer Beta-Brom-Azetamido-Funktion durch Kondensation mit N-Hydroxysuccinimidyl-Beta-Brom-Azetat aktiviert.

Durch entsprechende Funktionalisierungen an 3'- und 5'-Enden mehrerer Oligonukleotide und durch Führung der oben genannten Schritte lassen sich größere Oligonukleotidstücke herstellen, die beliebig viele funktionalisierte Beta-Brom-Azetamidogruppen aufweisen, welche potentielle Verzweigungspunkte darstellen. Aktivierbare Aminogruppen können auch im Rahmen einer Phosphoramiditsynthese direkt mit Hilfe von Aminoalkylphosphitderivaten in den Oligonukleotidstrang eingebaut werden. Dies hat den Vorteil, daß die Primärsequenz mit potentiellen Verzweigungspunkten an einem CPG-Träger durchgehend synthetisiert werden kann. In einem darauffolgenden Schritt wird ein drittes Oligonukleotid, welches unter Einbau von mittels einer markierungsvermittelnden Gruppe funktionalisierten Nukleotiden hergestellt wurde, am 5'-Ende in den Thiophosphatester umgewandelt. Durch Reaktion dieses Oligonukleotids mit den eingebauten Beta-Brom-Azetamido-Funktionen lassen sich nun auf direktem Wege (ohne Matrizen) verzweigte Multimere herstellen, welche über die Anzahl der Verzweigungspunkte und die Anzahl detektierbarer Markierungen pro Verzweigung die Basis für die gewünschte Signalamplifikation darstellen. Im Gegensatz zu den in WO 95/01365 beschriebenen Verfahren können in das erfindungsgemäße Verfahren relativ kurze dritte Oligonukleotide eingebaut werden. Wie oben beschrieben sind diese bevorzugt bereits bei ihrer Anknupfung an die Verzweigungspunkte durch eine markierungsvermittelnde Gruppe markiert, z B wahrend ihrer Synthese uber das Phosphoramiditverfahren (z. B nach EP-A-0 399 330)

Die markierungsvermittelnde Gruppe wird bevorzugt indirekt nachgewiesen. Sie wird bevorzugt mittels eines Konjugats aus eines zur markierungsvermittelnden Gruppe affinen Restes und einer signalgebenden Komponente nachgewiesen Solche affinen Reste sind beispielsweise Gruppen, die immunologisch mit der markierungsvermittelnden Gruppe unter Bindung des Konjugates an die markierungsvermittelnde Gruppe reagieren. Im Falle von Haptenen konnen daher Antikörper, die gegen dieses Hapten gerichtet sind, als affine Gruppen eingesetzt werden Für den Fall, daß das Hapten Digoxigenin ist, stehen für die Bildung von Konjugaten Antikorper gegen Digoxigenin zur Verfügung

Signalgebende Komponenten im Sinne der Erfindung sind Gruppen, die entweder direkt nachgewiesen werden konnen, oder aber bevorzugt, solche, die in einer chemischen Reaktion in eine nachweisbare Komponente uberfuhrt werden konnen. Besonders bevorzugte signalgebende Komponenten sind relativ kleine Proteine, besonders die kalziumaktivierbaren Photoproteine Unter dem Begriff Ca²⁻-aktivierbare Photoproteine wird eine Familie von lumineszenzfähigen molekularen Systemen zusammengefaßt, die folgende gemeinsame Merkmale aufweist

### a. physico-chemische Aspekte

- fertig ausgebildeter Reaktionskomplex aus einem proteinösen Katalysator (= Apo-Photoprotein), einem organisch-chemischen Substrat, welches fest aber nicht kovalent gebunden ist und den eigentlichen Emitter darstellt (= Luminophor), und ebenfalls fest fixiertem molekularem Sauerstoff (vermutlich kovalent protein-gebunden als Hydroperoxid), der erforderlich ist fur die Inituerung der Lumineszenzreaktion (= Oxidans)
- Unterscheidung von Enzymsystemen durch Gebundensein aller für die Lumineszenzreaktion notwendigen Komponenten
- relative Molmasse im Bereich 22000 Dalton
- "Coelenterazin" ([2-(p-Hydroxybenzyl)-6-(p-hydroxyphenyl)-8-benzyl-7-hydro-imidazopyrazin-3-on] als Luminophor
- Emissionspeakwellenlänge (λmax) im Blaubereich (ca 470 nm)
- Auslösung der Lumineszenzreaktion durch Bindung von 2-3 Ca²⁺-Ionen an das Apo-Photoprotein
- Vorhandensein von Ca²⁺-Bindedomanen im Apo-Photoprotein in Form von EF-Hand- (= Helix-Schleife-Helix-)strukturen
- Unabhängigkeit der Lumineszenzreaktion von Sauerstoff aus der Umgebung, d.h. das komplette Photoprotein luminesziert in Gegenwart wie auch in Abwesenheit von O₂ in der umgebenden Atmosphäre
- Photoprotein-Reaktion verlauft in Form einer Blitzkinetik, d.h. konzertierte Lichtemission als Folge von Ca²⁺-lonen-Zugabe
(siehe hierzu Blinks J R , et al , Pharmacological Reviews 28/1, 1-93, 1976)

### b molekularbiologische Aspekte

- funktionstaugliche Apo-Photoproteine mit einer Peptidlänge von i.d.R 189-196 Aminosäuren
- hohe Übereinstimmung der Nucleobasen- und Aminosäuresequenzen (> 60% Homologie) der einzelnen Vertreter der Familie der Ca²⁺-aktivierbaren Photoproteine, wie es die Klonierung von Aequorin (Prasher D , et al . Biochemical and Biophysical Research Communications 126/3, 1259-1268, 1985, Prasher D., et al , Methods in Enzymology 133, 288-299, 1986, Prasher D , et al , Biochemistry 26, 1326-1332, 1987; Cormier M., et al., Photochemistry and Photobiology 49/4, 509-512, 1989), Obelin (Illarionov Boris A., et al., Gene 153, 273-274, 1995), Clytin (Inouye S & Tsuji F., FEBS 315/3, 343-346, 1993) und Mitrocomin (Fagan T F , et al., FEBS 333/3, 301-305, 1993) deutlich gemacht hat.

Aus dieser Familie von Ca²⁺-aktivierbaren Photoproteinen hat in den letzten Jahren vor allem das Aequorin Anwendung im Bereich Rezeptor-Ligand-Bindungsassays gefunden. Bevorzugte Proteine sind beispielsweise Obelin, Halistaurin (= Mitrocomin), Phiallidin (= Clytin) oder Aequorin. Diese Proteine haben die Eigenschaft, daß sie ein Lichtsignal abgeben, wenn sie aktiviert werden, so daß ihre Menge oder Anwesenheit durch Messung der Blitzintensitat besummt werden kann. Die Verwendung solcher Photoproteine in herkömmlichen Tests und der Mechanismus, der zur Signalbildung fuhrt, ist beispielsweise in Cormier, M.L. et al , Photochem & Photobiol 49/4, 509 - 512 (1989) oder Smith, D.F et al., in "Bioluminescence and Chemiluniescence: Current Status (P. Stanley & L. Krick, eds.), John Wiley and Sons, Chichester, U.K. 1991, 529 - 532 beschrieben. Vorteile dieser signalgebenden Komponenten sind kürzere Meßzeit, erhöhte Quantenausbeute gegenüber anderen Lumineszenzmarkierungssystemen und erniedrigter reagenzbedingter Background, d. h. besser ausnutzbarer technischer Meßbereich der Luminometer bzw. breiterer dynamischer Meßbereich. Durch den relativ geringen Raumbedarf der kalziumaktivierbaren Photoproteine und ihrer Kofaktoren wird die Größe der nach Bindung des Konjugates entstehenden Sondenkomplexes stark reduziert. Außerdem interagieren solche kalziumaktivierbaren Photoproteine nicht mit DNA-Strukturen, wie es z. B. bei Acridiniumsalzen oder verschiedenen Chromophoren der Fall ist.

Durch Kombination aus minimaler unspezifischer Bindung von kleinen Aequorinkonjugaten, minimalem chemischen Rauschen von Label und Trigger, hoher spezifischer Photoproteinaktivität sowie sehr präziser Meßung der Aequorinlichtblitze auf hohem Signal-/Rauschniveau kann die gegenüber Enzymmarkierungen fehlende enzymatisch katalysierte Signalmultiplikation überraschenderweise, und entgegen der allgemein verbreiteten Meinung, mehr als ausgeglichen werden.

Die signalgebende Komponente und die zur markierungsvermittelnden Gruppe affine Komponente sind bevorzugt über einen gut solvatisierbaren Linker mit einer Länge von mindestens 4, bevorzugt mindestens 8 Atomen verknüpft. Dies bedeutet eine Erhöhung der Konformationsfreiheitsgrade und somit weniger sterische Hinderung. Dies wiederum kann bedeuten, daß die zugrundeliegenden Bindungsreaktionen schneller ablaufen können. Ein Kern der Erfindung ist, daß der Abstand zwischen benachbarten, an die Sonde gebundenen signalgebenden Komponenten, bei der vorliegenden Erfindung sehr klein sein kann. Dies wird insbesondere dadurch erreicht, daß im Stand der Technik verwendete identische Nukleotidsequenzen durch mit einer markierungsvermittelnden Gruppe markierte Mononukleoside ersetzt werden. Der Abstand zwischen den Mittelpunkten zweier auf einem Nukleotidstrang benachbarter markierungsvermittelnder Gruppen ist bei der Erfindung kleiner als 18 Nukleotide, bevorzugt kleiner als 15 Nukleotide, jedoch bevorzugt größer als 3 Nukleotide.

Der Abstand zwischen Verzweigungspunkten bei chemisch-kovalent verzweigten multimeren Molekülen ist bevorzugt kleiner als 7 Nukleotide; beträgt jedoch bevorzugt mindestens 1 Nukleotid.

Die markierungsvermittelnde Gruppe ist bevorzugt über einen Spacer, d. h. ein Brückenglied von 4 oder mehr Atomen, verknüpft. Die markierungsvermittelnden Gruppen können bei verzweigten Sonden, d. h. Sonden, die eine zentrale Primärsequenz mit mehreren Verzweigungspunkten und an die Verzweigungspunkte geknüpfte periphere Sekundärsequenzen enthalten, entweder in der Primär- oder der Sekundärsequenz oder in beiden Sequenzen eingebaut sein. Bevorzugt sind sie in beiden eingebaut.

Das erfindungsgemäße Verfahren läßt sich ganz besonders gut bei Nukleinsäuretests einsetzen, d. h. wenn der Analyt eine Nukleinsäure ist. Die Vorteile des erfindungsgemäßen Verfahrens zeigen sich jetzt auch schon bei ganz einfachen Verfahren, z. B dem Nachweis von Molekülen an einer Oberfläche, z. B. von Streptavidin, welches an die Oberfläche einer Tubewand gebunden ist. In diesem Fall kann als Sonde ein Oligonukleotid eingesetzt werden, welches als analytspezifischen Teil ein Biotinmolekül gebunden enthält. Der Oligonukleotidteil ist der Teil, der Nukleobasen an einem Backbone enthält, an den markierungsvermittelnde Gruppen, z. B. Digoxigenin, gebunden sind. Im Verfahren wird in das Tube mit streptavidinbeschichteter Oberfläche eine Lösung eingefüllt, die die Sonde im Überschuß über die Biotinbindungstellen des Streptavidin enthält. Nach Inkubation wird der Überschuß als Sonde entfernt und ein Konjugat aus Antikörper gegen Digoxigenin und Aequorin im Tube mit der immobilisierten Sonde inkubiert. Ein Überschuß des Konjugats wird ebenfalls wieder entfernt. Anschließend werden die für die Bestimmung des Aequorinlabels erforderlichen Reagenzien zugegeben und nach Triggerung der Lichtblitz gemessen. Aus der Blitzintensität ergibt sich die relative Menge an Aequorin und somit an gebundener Sonde und somit an Streptavidin an der Oberfläche.

Ein Vorteil des erfindungsgemäßen Verfahrens ist, daß die Sonde bei gleichbleibender Signalstärke sehr viel kleiner sein kann als bei Konzepten mit signalvermittelnden Hybridisierungszonen. Hierzu ist zu bemerken, daß die Löslichkeit verzweigter Nukleinsäuren mit zunehmender Größe sehr stark abnimmt. Dadurch, daß die erfindungsgemäßen Sonde bei gleichbleibender Anzahl von markierungsvermittelnden Gruppen deutlich kleiner sein kann als die bisher bekannten Sonden, kann entweder eine höhere Löslichkeit oder aber, bei gleichbleibender Molekülgröße und Löslichkeit, eine erhöhte Markierungsdichte erreicht werden. Es hat sich gezeigt, daß bei Verwendung nicht-nukleosidischer Hapten-Gruppen und relativ kleinen signalgebenden Komponenten und somit Konjugaten (bevorzugt kleiner als 100 KD) eine deutlich bessere Stöchiometrie der Bindung der Konjugate an die Sonde erreicht werden kann, d.h. die eingeführte Mehrfachmarkierung wird funktionell auch leichter zugänglich. Gründe hierfür sind zum einen weniger Raumerfüllung als bei enzym-(z. B. alkalische Phosphatase-, β-Galataktosidase-)markierter Detektions-Oligomersonden, zum anderen mehr Konformationsfreiheitsgrade, d h. flexiblere räumliche Orientierung durch geschickte Spacerung zwischen Hapten und Backbone wie auch zwischen anti-Hapten-Komponente und signalgebender Komponente des Konjugats. Dies erleichtert die Quantifizierung der Analyse. Zudem erfolgt bei einem solchen Hybridisierungsimmuno-assay, im Gegensatz zu einem reinen Hybridisierungsassay, die Labet- bzw. Konjugatinkubation bei vergleichsweise moderaten Temperaturen, z B 37 °C, was erhebliche Vorteile in puncto Retention der zugesetzten spezifischen Aktivitat des Konjugates bedeutet. Bei einem reinen Hybridisierungsassay laufen hingegen die Anforderungen bezuglich Spezifität (Temperatur so hoch wie moglich) und bezüglich Labelaktivitat (Temperatur eher niedrig) einander diametral zuwider. Thermische Destruktion von Konjugatfunktion reduziert unmittelbar die erreichbare analytische Testsensitivitat Die Vielfachmarkierung der erfindungsgemaßen Sonde wird nach dem erfindungsgemäßen Verfahren praparativ leichter zugänglich. Zusatzlich konnen nach dem erfindungsgemaßen Verfahren im Gegensatz zu signalvermittelnden Hybridisierungszonen, Markierungen auch ohne signifikante Molekül-vergrößerung in die Primarsequenz des Backbone eingebaut werden Das erfindungsgemäße Verfahren ist auch für Therapiekontrolle geeignet.

In Figur 1 ist ein Komplex gezeigt, der im Laufe eines erfindungsgemäßen Verfahrens zum Nachweis einer Nukleinsäure gebildet werden kann

In Figur 2 ist ein Verfahren zum Nachweis von Ribonukleinsäuren aus Zellen beschrieben.

In Figur 3 ist ein Verfahren zum Nachweis von DNS aus Zellen beschrieben .

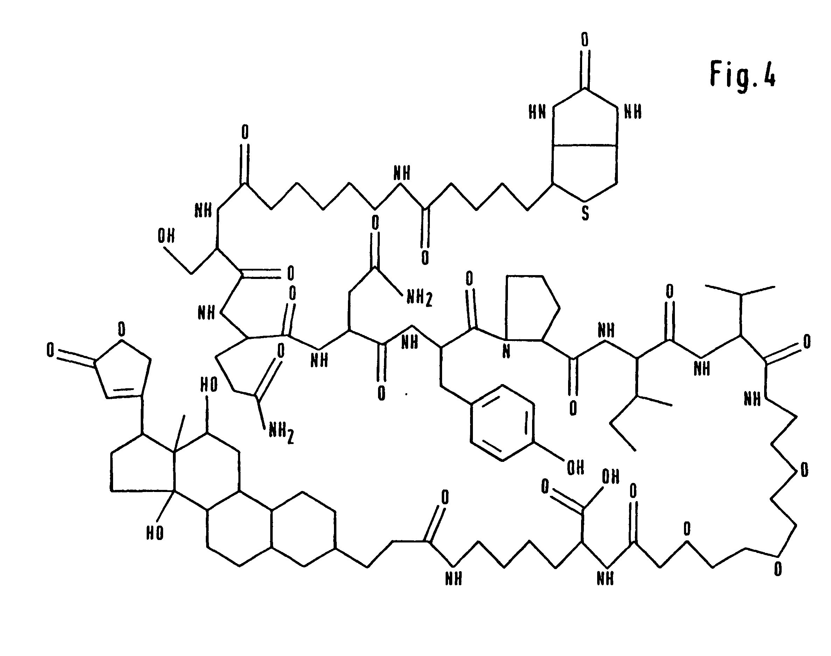

In Figur 4 ist die Struktur eines in den Beispielen verwendeten Peptids (MDP) gezeigt.

Figur 5 zeigt schematisch den Aufbau von Oligonukleotid 1, eines verzweigten Oligonukleotids gemäß der vorliegenden Erfindung.

Figuren 6-11 zeigen die Sequenz weiterer Oligonukleotide, die entweder als Zwischen- oder Endprodukte gemäß der vorliegenden Erfindung eingesetzt werden können.

Figur 12 zeigt die Struktur eines in den Beispielen verwendeten Reagenzes zur Einführung der Markierungsgruppen.

Figur 13 zeigt vergleichende Signalmessungen für die hergestellten und in Verfahren zum Nachweis von Nukleinsäuren eingestzten Oligonukleotide.

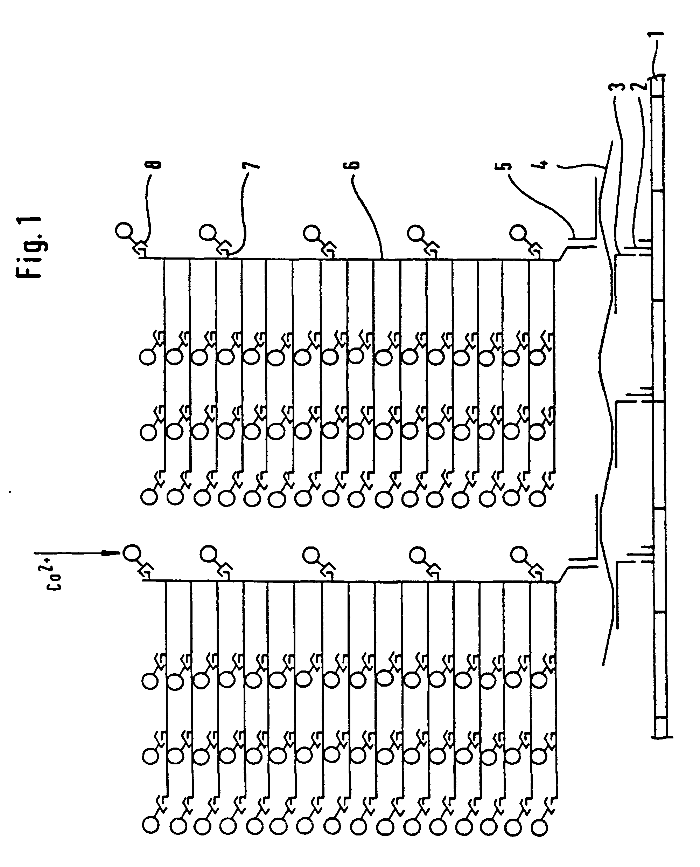

In Figur 1 ist erkennbar, daß die nachzuweisende Nukleinsäure (4) über sogenannte Fangsonden (3) an eine universelle Festphase, bestehend aus z. B. biotinylierter Festphasensonde und (Strept)Avidin-beschichteter Testträgerwand, gebunden werden konnen. Alternativ dazu kann auf eine spezielle Festphasensonde verzichtet und der Reaktionskomplex direkt über biotinylierte Fangsonden immobilisiert werden. Die solchermaßen gebundene nachzuweisende Nukleinsäure wird über eine vermittelnde Sonde (5) und die erfindungsgemäßen Sonden (6) an der festen Phase nachgewiesen. Die Bindung der Nukleinsäure an die feste Phase kann an mehreren Stellen gleichzeitig geschehen. Die Bindung der erfindungsgemäßen Sonde an die nachzuweisende Nukleinsäure kann ebenfalls mehrfach geschehen. Es ist hier von Vorteil, wenn nur eine einzige Art von Sonden zum Nachweis verschiedener Nukleinsauren verwendet werden muß. Hierbei spielen die Zwischensonden (5) eine die Universalität sicherstellende Rolle Die in Figur 1 gezeigte Sonde ist aufgebaut aus einer Primärsequenz, welche 16 Verzweigungspunkte und daran gebunden 16 Sekundärsequenzen aufweist. In die Primärsequenz sind in Bereichen, die nicht mit den Verzweigungspunkten überlappen, nicht-nukleosidische, markierungsvermittelnde Gruppen (7) eingebaut. Darüber hinaus enthält jede der Sekundarsequenzen 3 markierungsvermittelnde nicht-nukleosidische Gruppen. Aufgrund der sterischen Bedingungen der erfindungsgemäßen Sonde ist eine nahezu stöchiometrische Absättigung der nicht-nukleosidischen, markierungsvermittelnden Gruppen durch Bindung von Konjugaten aus einem gegen die markierungsvermittelnde Gruppe gerichteten Antikörper und einem kalziumabhängigen Photoprotein gefördert.

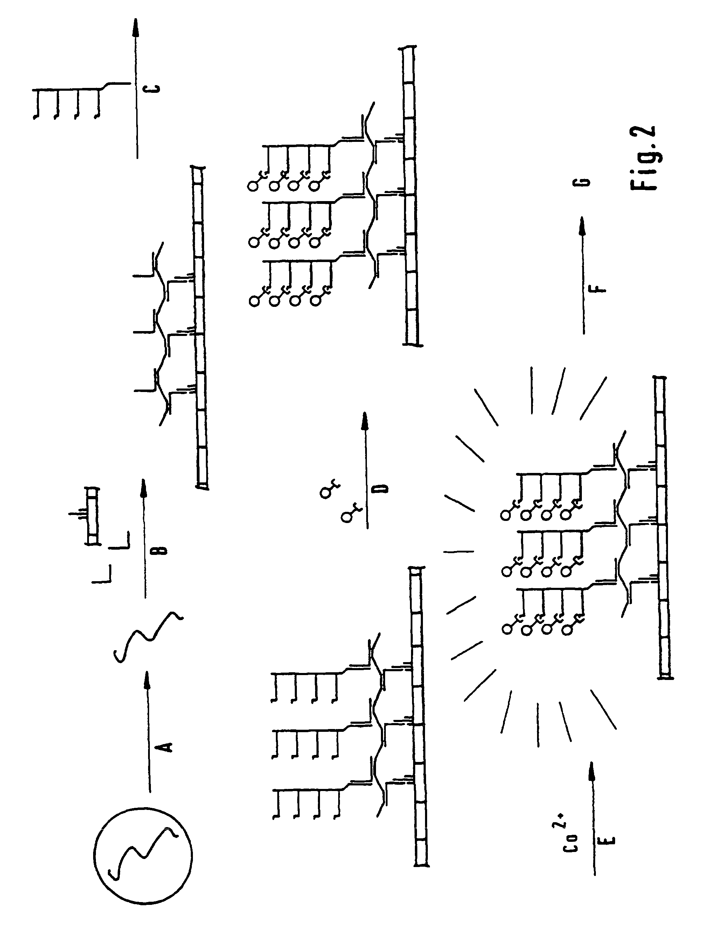

In Figur 2 ist der Ablauf eines Bestimmungsverfahrens für eine Ribonukleinsaure (RNA) aus einer Zelle schematisch gezeigt. Zu Anfang des Verfahrens liegt die Ribonukleinsäure in der Zelle vor. In einem ersten Schritt A wird die Zelle aufgebrochen (nach bekannten Verfahren, z. B. Lyse mit Hilfe von Proteasen), eventuell vorhandene RNAsen abgebaut und die RNA freigesetzt. In einem folgenden Schritt B werden die Oligonukleotide (5), die Fangsonden (3) und die RNA-haltige Probe in einer Mikrotiterplatte vereinigt und inkubiert. Hierdurch werden die Nukleinsäuren sowie die zugesetzten Sonden an die feste Phase gebunden. In einem Schritt C wird (nach Abwaschen nicht-gebundener Probenbestandteile und überschüssiger Fangsonden sowie Oligonukleotiden (5)) erfindungsgemäße Sonden (6) im Überschuß über die zu erwartende Menge an gebunden Oligonukleotiden (5) unter Bedingungen inkubiert, bei denen der universelle Nukleotidanteil der Sonde mit dem universellen Anteil des Oligonukleotids (5) hybridisieren kann Ein Überschuß an nicht-gebundenen erfindungsgemäßen Sonden wird durch einen Waschschritt entfernt. In einem Schritt D werden die über die nachzuweisende Nukleinsaure an die Festphase gebundenen Sondenmoleküle bzw. die an ihnen befindlichen markierungsvermittelnden nicht-nukleosidischen Gruppen durch Zugabe eines Konjugats (8) und Inkubation besetzt. Auch hier wird ein Überschuß an Konjugat abgewaschen. In einem darauffolgenden Schritt E werden die für den Nachweis der signalgebenden Komponente erforderlichen Reagenzien, im speziellen Fall als Kalziumionen als Trigger zugegeben. Im Schritt F wird der entstandene Lichtblitz G als Meßsignal, angegeben in relative luminescence units [RLU] zur direkten Quantifizierung der Menge an nachzuweisender Nukleinsäure gemessen und ausgewertet .Da die Intensität des Blitzsignals direkt proportional zu der Anzahl der gebundenen Konjugate und somit der Menge an gebundenen nachzuweisenden Nukleinsäuren proportional ist, kann aus der Blitzintensität direkt die Menge an Nukleinsäure ermittelt werden.

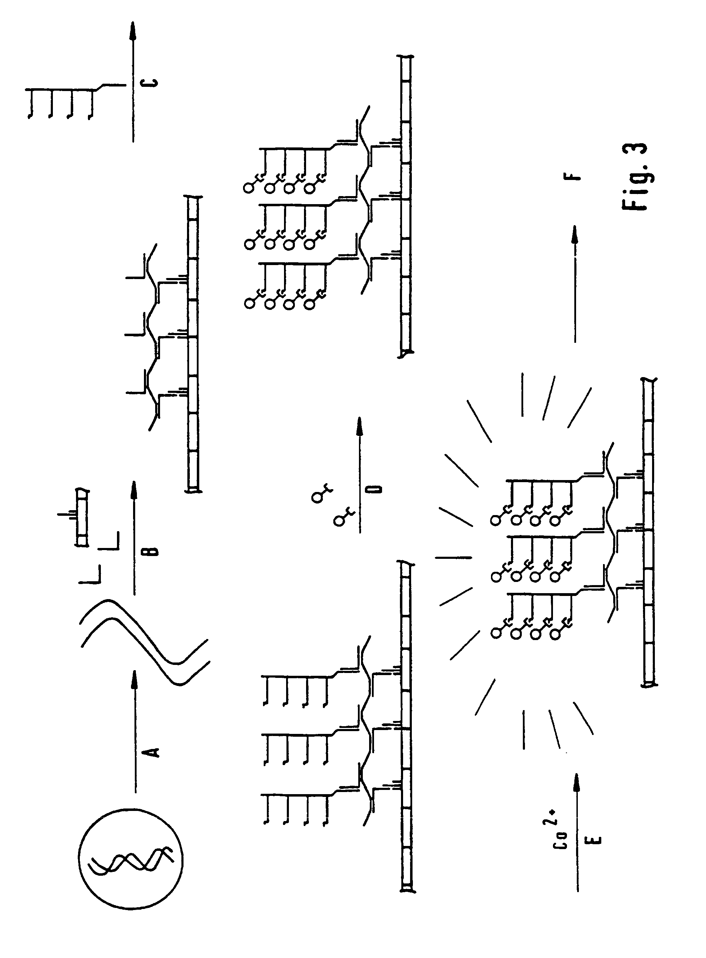

In Figur 3 ist dasselbe Verfahren, jedoch unter Benutzung von DNA als nachzuweisende Nukleinsäure schematisch gezeigt. Auch hier werden die Zellen zunächst aufgebrochen, um die DNA freizusetzen. Der erste Schritt A enthalt darüber hinaus eine Denaturierung der eventuell doppelsträngig vorliegenden DNA .Dies kann beispielsweise durch einen Hitzeschritt oder unter alkalischen Bedingungen geschehen.

Bevorzugte Eigenschaften der Erfindung können folgende sein:
1. Die Amplifikationseinheit bildet sich nicht im Laufe des Tests via Hybridisierung / Aggregation einzelner Sonden, soridern ist ein definiertes, präformiertes Reagenz im Sinne einer chemischen Verbindung mit speziell geplanter Konstitution und Konfiguration.
2. Verwendet werden keine Hybridisierungszonen für nachfolgende Detektionssonden, sondern niedermolekulare Reportermoleküle in einem Abstand voneinander, der kleiner ist als übliche Längen von Hybridisierungszonen (16-26 Nukleotide), d. h. präparativ leichterer Zugang zu hoher Signalstärke bei gleichbleibender Molekülgröße.
3. Eingesetzt werden kleine, flexibel gespacerte Reportergruppen in Verbindung mit vergleichsweise kleinen, ebenfalls via Linker flexibel verknüpften Konjugaten, und kleinen signalauslösenden Triggersubstanzen ohne höhermolekulare Additive. Dies bewirkt eine bessere funktionelle Umsetzung durch sterische Vorteile in puncto Raumerfüllung und räumliche Orientierung.
4. Die hohe Reportergruppendichte stört eine eventuelle (Selbst-)Hybridisierungsfähigkeit des signalvermittelnden Teils, der Nukleobasen an einem Backbone enthält, der Sonde, d. h. keine speziellen Anforderungen an die Nukleotidsequenz oder diesbezügliche Symmetrie-Eigenschaften des signalvermittelnden Teils.
5. Verwendung des Prinzips eines Hybridisierungs-immuno-assay, d. h. bessere Retention der spezifischen Aktivität des Nachweisreagenz.
6. Die Verwendung einer Amplifikationssonde zusammen mit einem sterisch wenig anspruchsvollen, gegenüber DNA inerten Nachweissystem, welches hohe TestSensitivität nicht durch cyclische, zusätzliche Signalverstärkung, sondern sehr hohes Signal-Rausch-Verhältnis unterstützt, wie dem Aequorinsystem, ist besonders vorteilhaft.
7. Der technische Meßbereich der Luminometer ist somit voll ausnutzbar, d. h. es resultiert ein breiterer dynamischer Meßbereich.

Die folgenden Beispiele sollen die Erfindung näher erläutern:

### Beispiel 1

### Herstellung eines Konjugates aus einem polyklonalen Antikorper gegen Digoxigenin und Aequorin

Recombinantes Aequonn (AquaLite™. Fa SeaLite Sciences, Inc) wird in einer Konzentration von 2.4 mg/ml in Puffer der folgenden Zusammensetzung vorgelegt:
10 mM HEPES, 200 mM NaCl, 2 mM EDTA, 2 mM DTT, pH 8.0)

Durch Reaktion (30 min/25°C/Ruhren) mit einem 15fachen molaren Überschuß an 2-Iminothiolan (= Traut's Reagenz) werden ca 1 2 bis 1 6 zusatzhche SH-Gruppen eingeführt. Die Reaktion wird abgestoppt durch Zugabe von L-Lysin (≥ 15 min/25°C/Rühren, Endkonzentration 10 mM) und die Reaktionslösung umgepuffert in Konjugatpuffer und aufkonzentriert.

Durch Affinitätschromatographie (positive Immunsorption) aufgereinigtes Anti-DIG-Fab-Fragment (gewonnen aus Schaf-Antiserum) wird in einer Konzentration von ≥ 5 mg/dl in 30 mM Na-Phosphatpuffer pH 7 l gelöst und mit einem 1 3- bis 1.7fachen molaren Uberschuß an MHS (Maleinimido-hexanoyl-N-hydroxysuccinimidat) oder SMCC (Succinimidyl-4-[N-maleimidomethyl)-cyclohexan-1-carboxylat), gelöst in DMSO, umgesetzt (60 min/25°C/Ruhren). Hierdurch wird ca.eine MH-Gruppe pro Fab-Fragment eingebaut. Die Reaktion wird abgestoppt durch Zugabe von L-Lysin (30 min/25°C/Rühren, Endkonzentration 10 mM) und die Reaktionslösung umgepuffert in Konjugationspuffer und aufkonzentriert.

Die beiden aktivierten Komponenten werden schließlich in einer Kopplungsstöchiometrie von Aequorin-SH: Fab-MH = 2.5-1 in 25 mM HEPES, 3 mM EDTA, pH 7 5 zur Reaktion gebracht. Dabei beträgt die Konzentration von Aequorin-SH ca. 1 mg/ml. Die Reaktion erfolgt fur 60 min bei 25°C unter Rühren. Der Reaktionsstop erfolgt durch Zugabe von Cystein bzw. Dithiothreitol (2 mM bzw 5 mM Endkonzentration).

Das entstandene <DIG>-Fab-Aequorin-Konjugat wird schließlich aufgereinigt durch eine Kombination aus Trennung nach Ladung (Ionenaustauschersäule, Q Sepharose FF oder DEAE 5PW) und Trennung nach Molekülgröße (Gelfiltrationssäule, Sephacryl S 200-HR), eingeengt und in einem HEPES-Lagerpuffer bei -70°C aliquotiert gelagert.

Die Reihenfolge der Auftrennungsschritte kann umgedreht werden.

Zu den Versuchen werden Aliquots aufgetaut, in Testpuffer auf Arbeitslösungskönzentration verdünnt und noch am selben Tag eingesetzt.

### Beispiel 2

### Herstellung einer unverzweigten Sonde (TDN)

Herstellung eines Oligonukleotids der Formel mit X = Biotinhexapropanolphosphat und
Y = 3-Amino-1,2-Propandiolphosphat, wobei Y nachträglich mit Digoxigenin-3-O-methylcarbonyl-6-aminocapronsäure-N-hydroxy-succinimidester markiert wird.

Die Synthese erfolgt auf einem Gene Assembler Plus der Firma Pharmacia nach den Standardprotokollen der Bedienungsanleitung. Als Nukleotidbausteine werden die Standardphosphoramidite 5'-Dimethoxytrityl-N-benzoyl-2'-deoxy Adenosin, 3'-[(2-cyanoethyl)-(N,N-diisopropyl)]-phosphoramidit, 5'-Dimethoxytrityl-N-benzoyl-2'-desoxy Cytidin, 3'-[(2-cyanoethyl)-(N,N-diisopropyl)]-phosphoramidit, 5'-Dimethoxytrityl-N-isobutyryl-2'-desoxy Guanosin, 3'-[(2-cyanoethyl)-(N,N-diisopropyl)]-phosphoramidit und 5'Dimethoxytrityl-2'-desoxy Tymidin, [(2-cyanoethyl)-(N,N-diisopropyl)]-phosphoramidit (der Firma ABI) verwendet. An der X-Position wird das Biotinphosphoramidit (ABI 401395) und an der Y-Position das 9-Fluorenyl-methoxycarbonyl-3-Amino-1-Dimethoxytrityl-2-Propandiol-[(2-cyanoethyl)-(N,N-diisipropyl)]-phosphoramidit eingesetzt. Der Synthesemaßstab ist 1,3 µmol auf einem 5'-Dimethoxytrityl-2'-desoxy Thymidin 3-succinoyl-long chain alkylamino Controlled Pore Glass Trager Nach 20 Stunden bei 37°C in einer 32%igen Ammoniaklosung wird das Oligonukleotid vom Träger und gleichzeitig die β-Cyanoethyl-Gruppen sowie die Schutzgruppen der freien Aminoreste abgespalten. Das Rohprodukt wird durch HPLC (Lichrosorb RP 18.8 x 250 mm, von CS-Chromatographie Service) uber einen 0.1 m Triethylammoniumacetat/Acetonitril Gradienten gereinigt und mittels Dialyse entsalzt. Nach Entfernung des Lösungsmittels im Vakuum wird das Oligonukleotid gemaß dem Boehringer Mannheim Protokoll (EP 0 371 262) mit dem Digoxigenin-3-O-methylcarbonyl-5-aminocapronsäure-N-hydroxysuccinimidester gelabelt. Nach der Markierungsreaktion wird das Oligonukleotid dialysiert, aufkonzentriert und chromatographisch (HPLC Lichrosorb RP 18. 8 x 250 mm, CS-Chromatographie Service) uber einen 0.1 M Triethylammoniumacetat/Acetonitril Gradienten gereinigt und mittels Dialyse entsalzt. Der Nachweis der Digoxigenin-Markierung erfolgt nach Gebevechu. G etal (1987), Nucl Acids Res 15, 4513

### Beispiel 3:

### Nachweis von Streptavidin an einer Mikrotiterplatte mit Hilfe des erfindungsgemäßen Verfahrens und Vergleich mit einfachmarkierten Sonden

| Testsensitivitat mit Anti-DIG-Fab-Aequorin-Konjugat | |
|---|---|
| Testträger | weiße Mikrotiterplatten von der Fa. Nunc, Typ MaxiSorb, bei der Fa MicroCoat mit tRSA-SA (thermo RSA-Streptavidin) beschichtet |
| Volumina. | 25 µl DIG-Reagenz (Mono-Biotin-mono-Digoxigenin-Heptapeptid, FIG 4, (MDP), in verschiedenen Konzentrationen) + 50 µl <DIG>-Aequorin-Konjugat aus Beispiel I in Testpuffer (ca 4 x 10⁶ RLU / Test) |
| Testformat | 15 min Simultaninkubation (I-Schritt-Assay) |
| b/f-Trennung | SLT plate washer SW 812 A2, Prog 2 (= 4 x Waschen) |
| Testpuffer | 25 mM HEPES, 150 mM Kcl, 10 mM EGTA. 2 mg/ml RSA, 1 5 mg/ml RSA-c, 0 05 % (v/v) Tween-20, 0 1 % (w/v) NaN₃, pH 7.0 |
| Meßgerat | Dynatech ML 3000 plate reader, Peak-Modus (0 1 sec vor Peak- + 2 0 sec nach Peak-Intervall) |

| pmol/l DIG-Reagenz | gebundenes Signal [RLU] (Mittelwert ± Standardabweichung) | Steilheit (S/N) |
|---|---|---|
| 0 0 | 3346 ± 457 | |
| 0 2 | 9079 | 2 71 |
| 2 0 | 53405 | 15 96 |
| 20 0 | 611900 | 182.86 |
| 200 0 | 5290000 | 1581 00 |
| 1000 0 | 26400000 | 7890 00 |

unter Nachweisgrenze* = 0 032 pmol/l = 8 x 10⁻¹⁹ mol/Kavitat = ca 4 8 x 10⁵ Analvtmolekule
* berechnet auf Basis Nullwert + 2fach Standardabweichung aus Replikatmessung

### Beispiel 4:

Vergleichende Bewertung Mono-Biotin-mono-Digoxigenin-Heptapeptid (MDP) vs Mono-5'-Biotin-Tri-Digoxigenin-28mer-Oligonukleotid (TDN)

| | |
|---|---|
| Testtrager | weiße Mikrotiterplatten von der Fa Nunc, Typ MaxiSorb, bei der Fa. MicroCoat mit tRSA-SA (thermo RSA-Streptavidin) beschichtet |
| Volumina. | 25 µl DIG-Reagenz (Mono-Biotin-mono-Digoxigenin-Heptapeptid, in verschiedenen Konzentrationen) + 50 µl <DIG>-Aequorin-Konjugat aus Beispiel 1 in Testpuffer (ca 4 x 10⁶ RLU / Test) |
| Testformat. | 15 min Simultaninkubation (1-Schritt-Assay) |
| b/f-Trennung | SLT plate washer SW 812 A3, Prog 2 (= 4 x Waschen) |
| Testpuffer | 25 mM HEPES, 150 mM Kcl, 10 mM EGTA, 2 mg/ml RSA, 1.5 mg/ml RSA-c, 0 05 % (v/v) Tween-20, 0 1 % (w/v) NaN₃, pH 7 0 |
| Meßgerat | Dynatech ML 3000 plate reader, Integrationsmodus |

| gebundes Signal | | | | | |
|---|---|---|---|---|---|
| DIG-Reagenz [pmol/l] | [RLU]- TDN | | [RLU] - MDP | | Faktor TDN/MDP |
| | Exp 1 | Exp 2 | Exp 1 | Exp. 2 | |
| 0.0 | 378 | 383 | 143 | 119 | |
| | (170 | 182) | (200 | 196) | |
| 0 1 | 498 | -173 | 109 | 115 | |
| 1.0 | 1075 | 1150 | 323 | 289 | 3.33 |
| | | | | | 399 |
| 10.0 | 7055 | 7150 | 1895 | 1935 | 3 72 |
| | | | | | 3 70 |
| 100 0 | 60850 | 54350 | 19550 | 19250 | 3 11 |
| | | | | | 2 82 |

### Anmerkung.

Bei diesem Experiment wurden MDP und TDN nacheinander abgearbeitet. Aufgrund einer Nachvernetzungsreaktion in der Konjugat-Arbeitslosung kam es zu einer gewissen Aggregatbildung, bei TDN (spater¹) mehr als bei MDP, und damit zu unterschiedlicher unspezifischer Bindung, Dieser Unterschied ist aber zu gering, um die höheren spezifisch gebundenen Signalniveaus signifikant zu verfälschen.

In einem Folgeexperiment konnte gezeigt werden, daß bei Abarbeitung in einem Lauf die unspezifische Bindung für beide DIG-Reagenzien ca. identisch ist (siehe Nullwene für TDN und MDP in Klammern) In einem Zusatzexpenment mit nur mit Blocklösung beschichteten Platten konnte gezeigt werden, daß die unspezifische Bindung für beide DIG-Reagenzien über den ganzen Konzentrationsbereich hinweg praktisch identisch ist.

### Beispiel 5

### Herstellung eines verzweigten Oligonucleotids

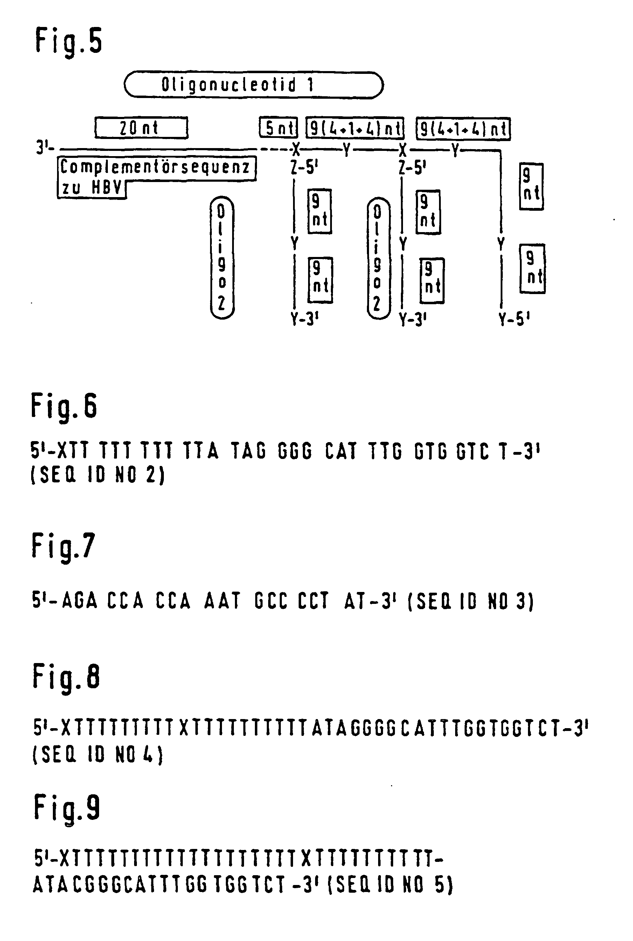

Zur Erläuterung dieses Beispiels wird Bezug genommen auf die Abbildungen (FIG 5-12). Hierbei bedeuten in FIG 5 X Verzweigungspunkte via 3-Amino-1,2-Propandiolphosphat, Y . Markierungspunkte via DNP-TEG-Phosphoramidit und Z Verknüpfung via Mercaptohexyl.

### 1. Synthese der Oligonucleotide

Für die einzelstrangigen und linearen Oligonukleotide sind die Sequenzen den Sequenzprotokollen zu entnehmen. Fur die einzelsträngigen, aber verzweigten Oligonukleotide sind die Sequenzen den Abbildungen zu entnehmen. Die Synthesen erfolgen auf einem Gene Assembler Plus der Firma Pharmacia nach den Standardprotokollen der Bedienungsanleitung und den Angaben der Phosphoramidithersteller.

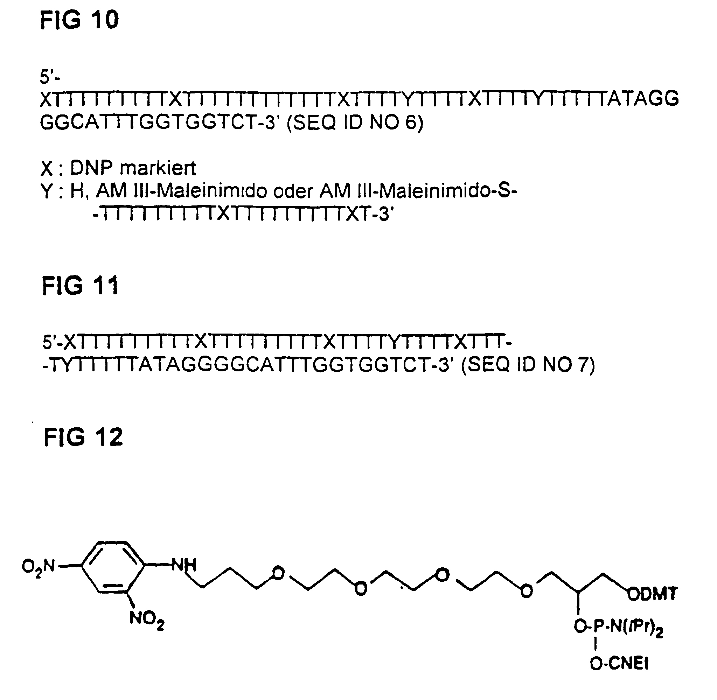

Als Nucleotid-bausteine werden die Standardphosphoramidite 5'-Dimethoxytrityl-N-benzoyl-2 -deoxy Adenosin. 3 -[(2-evanoethyl)-(N,N-diisopropyl)]-phosphoramidit, 5'-Dimethoxytrityl-N-benzoyl-2'-desoxy Cytidin, 3'-[(2-cyanoethyl)-(N,N-diisopropyl)]-phosphoramidit, 5'-Dimethoxytrityl-N-isobutyryl-2'-desoxy Guanosin, 3'-[(2-cyanoethyl)-(N,N-diisopropyl)]-phosphoramidit und 5'Dimethoxytrityl-2'-desoxy Thymidin,[(2-cyanoethyl)-(N,N-diisopropyl)]-phosphoramidit (der Firma ABI) verwendet An der X-Position wird 3-[N-(9-Fluorenylmethoxycarbonyl)-6-aminocapronsaure]-amino-1-(4,4'-dimethoxytrityl)-1,2-propandiol-2-[(2-cyanoethyl)-(N,N-diiso-propyl)] phosphoramidit zur Verzweigung, an der Y-Position das DNP-TEG-Phosphoramidit (FIG 12, Glen Research 10-1985-, siehe auch Nucleic acids Research 21, 1993, 1705-1712) zur Markierung und an derZ-Position der 5'-Thiol-Modifier C6 (Glen Research 10-1926-) zur Verknupfung verwendet. Der Synthesemaßstab ist 1.3 µmol auf einem 5'-Dimethoxytriryl-2'-desoxy Thymidin 3'-succinoyl-long chain alkylamino Controlled Pore Glass Träger Nach 5 Stunden bei 55 °C in einer 32%igen Ammoniaklösung werden die Oligonucleotide vom Träger und gleichzeitig die β-Cyanoethyl-Gruppen sowie die basenlabilen Amino-Schutzgruppen abgespalten. Die Rohprodukte werden durch HPLC (Lichrosorb RP 18, 8 X 250 mm, von CS - Chromatographie Service) uber einen 0.1 M Triethylammoniumacetat / Acetonitril Gradienten gereinigt und mittels Dialyse entsalzt. Die Thiol-Gruppe des Oligonucleotids 2 wird gemäß Glen Research User Guide entschutzt und danach 5 mal mit Essigester extrahiert und im Speedvac lyophilisiert.

Nach dieser Vorschrift wurden die Oligonucleotide 1XDNP (= 1Xlin), 2XDNP (=2Xlin) und 2X'DNP (=2'Xlin) sowie Oligonucleotid 1 und 2 synthetisiert.

### 2 Maleinimidofunktionalisierung des Oligonucleotid I

Das lyophilisierte Oligonucleotid 1 wird in 350 µl Natriumhydrogencarbonat-Puffer, 0,1mol/l, pH 8,5, gelost. Dazu gibt man eine Lösung von 1 mg Maleinimido-hexyl-N-hydroxysuccinimidester in 50 µl Acetonitril Die Reaktionslösung wird über Nacht im Thermomixer bei 20 °C geschüttelt Das Oligonucleotid wird über eine NAP-25 Saule (Pharmacia) vom freien Maleinimid und Salz abgetrennt, danach aufkonzentriert und chromatographisch (HPLC Lichrosorb RP 18, 8 X 250 mm, CS - Chromatographie Service) uber einen 0, 1 M Triethylammoniumacetat / Acetonitril Gradienten gereinigt und mittels Dialyse entsalzt.

### 3 Verknüpfungsreaktion von Oligonucleotid 1 und 2

10 OD von Oligonucleotid 1 wird in 50 µl 0.1 M Phosphatpuffer pH 6,0 gelost und zu 30 OD Oligonucleotid 2 gegeben Die Reaktionslosung wird 3 Tage im Thermomixer bei 20 °C geschüttelt. Die Reinigung erfolgt uber ein 8 %iges praparatives Polyacrylamid Gel, wobei von dem gewünschten 8XDNP-Oligonucleotid (=8XbNA) Eduktoligonucleotide und insbesondere das 6XDNP-Zwischenprodukt (=6XbNA) abgetrennt wurde. Das 6XDNP-Oligonucleotid resultiert aus nur einfacher Kopplung von Oligonucleotid 2 an Oligonucleotid 1. Man erhalt 6.6 OD 8XDNP-Oligonucleoud und 4,8 OD 6XDNP-Oligonucleoud Die Reinheit wurde mittels HPLC (RP 18 Hypersil ODS 4,5 X 250 mm von CS - Chromatographie Service) über einen 0.1 M Triethylammoniumacetat / Acetonitril Gradienten bestimmt.

### Beispiel 6

### Hybridisierungsimmunoassay mit DNP-markierten Indikatorsonden

Die unter Beispiel 5 beschriebenen Indikatorsonden wurden nun vergleichend in Hybridisierungsimmunoassays ausgetestet

### Hierbei bedeuten

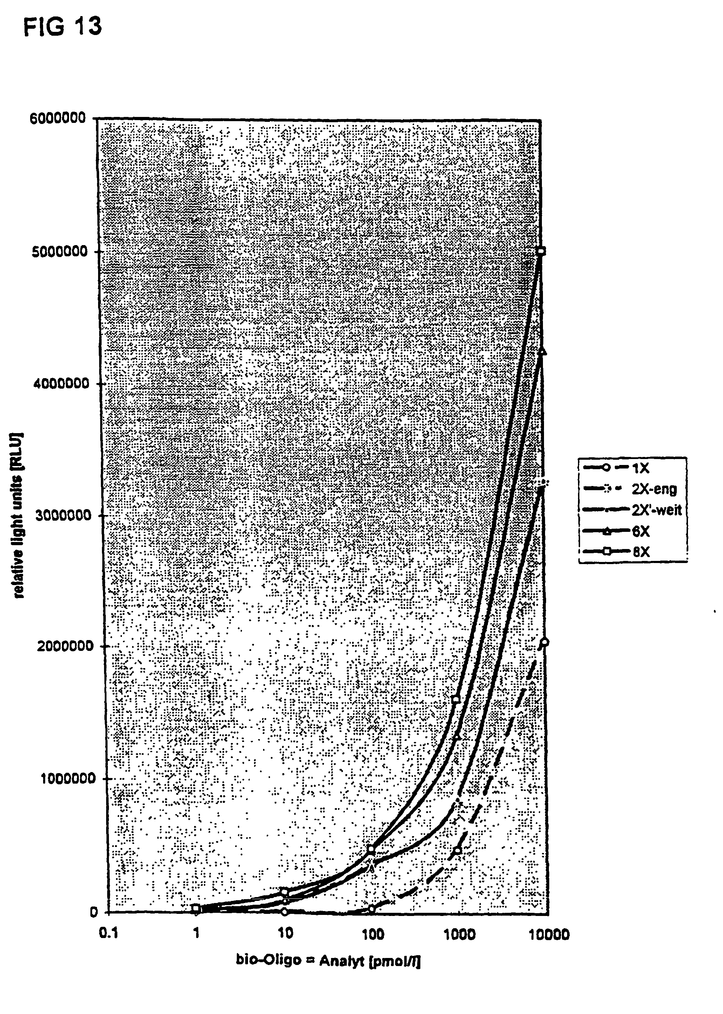

- 1X lin = lineares 31 mer DNA-Ohgo mit einer DNP-Markierung 5'-terminal und einer zu dem HBV-Oligo #33-1 (FIG 7) complementären 20nt-Sequenz 3'-terminal
- 2X lin (auch 2Xeng in FIG 13) = lineares 41mer DNA-Oligo mit zwei DNP-Markierungen (Pos 1 und 11. 5'→3', d h durch einen 9nt-Spacer voneinander getrennt), und einer zu dem HBV-Oligo #33-1 complementaren 20nt-Sequenz 3'-terminal
- 2X' lin (auch 2Xweit in FIG 13) = lineares 51mer DNA-Oligo mit zwei DNP-Markierungen (Pos 1 und 21, 5'→3', d h durch einen 19nt-Spacer voneinander getrennt), und einer zu dem HBV-Oligo #33-1 complementaren 20nt-Sequenz 3'-terminal
- 6X bNA = verzweigtes (branched) 83mer DNA-Oligo mit insgesamt 6 DNP-Markierungen, 2 in der Stammsequenz und je 2 in den beiden Zweigsequenzen, gemäß FIG 10
- 8X bNA = verzweigtes (branched) 104mer DNA-Oligo mit insgesamt 8 DNP-Markierungen. 2 in der Stammsequenz und je 2 m den drei Zweigsequenzen, gemaß FIG 11

Als Immobilisierungsreagenz und zugleich nachzuweisender Analyt dienten verschiedene Konzentrationen eines zur HBV-Wildtyp-Sequenz complementaren, 5'-biotinylierten 20 nt-Oligomers

### Testaufbau

- Testtrager weiße MaxiSorp™ Mikrotiterplatten der Fa. Nunc, SA-beschichtet, bzw weiße unbeschichtete MicroLite™ Mikrotiterplatten der Fa. Dynatech.
- Immobilisierung via 20nt-Oligo-5'-Biotin (FIG 7) im Konzentrationsbereich 0, 1 - 10000 pmol/l. welches zugleich als nachzuweisender Analyt fungiert.
- Testpuffer für Hybridisierungsexperimente
   25 mM Hepes pH 7 4
   150 mM NaCl
   10 mM EGTA
   0 5% (w/v) RSA
   0 5% (w/v) Tween-20
   0 1% NaN₂

   Optionale Zusatzadditive 0 15 (v/v) RSA-c (teilweise linearisiertes und acetyliertes RSA-Praparat von der Fa Aurion/NL. bzw 200 mM Na₂-Tartrat x 2H₂O
- Testformat 3-Schritt delayed solid phase-Assay
- Testschritte Hybridisierung in Losung von 20nt-Oligo-Biotin und jeweils einer der beschriebenen DNP-markierten Indikatorsonden für 60 min/RT/Schüttein in unbeschichteten Mikrotiterplatten, danach Transfer in SA-beschichtete Mikrotiterplatten via Multikanalpipette und Inkubation fur 15 min bei RT/Schutteln zwecks Immobilisierung via SA-Biotin-Wechselwirkung, Waschen (d h. Trennung von festphasengebundenen und ungebundenen Reaktionskomplexen), und abschließend 30 min Inkubation bei RT/Schütteln zwecks Reaktion der festphasengebundenen Hybridisierungskomplexe mit einem MAK<DNP>M-Fab-Aequorin-Konjugat (hergestellt analog dem <DIG>-Aequorin von Beispiel 1). Nach abermaligem Waschen erfolgt die Messung der Aequorin-Biolumineszenz, die Signalstärke ist der Analytkonzentration direkt proportional
- Reaktionsvolumen 50 µl 20nt Oligo-5'-Biotin (= Analyt) + 50 µl Indikatorsonde, davon Transfer von 50 µl Reaktionslosung pro Kavitat; 50 µl Konjugat
- Waschmodul SLT Columbus Plate Washer
- Meßmodul Dynatech ML 3000 Plate Luminometer
- Trigger 10 mM Tns, pH 7 4, mit 100 mM CaCl₂
- Meßmodus integrated flash mode. 0 I sec before peak + 1 0 sec after peak, gain high

### Beispiel 6 A

Unter den genannten Testbedingungen wurde hier ein Konzentrationsbereich an 20nt-HBV-Oligo-5'-Biotin von 0 - 1000 pmol/l vermessen Die DNP-markierten Sonden wurden in einer Konzentration von 10 nmol/l eingesetzt, das <DNP>Aequonn-Konjugat in einer Konzentration von ca 1 x 10⁶ RLU /Test Die Ergebnisse zeigt Tabelle 1

**Tabelle 1**

| **DNA-Hybridisierungsimmunoassay mit DNP-markierten Indikatorsonden** | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 3,38E+03 | 5,27E+03 | 4,02E+03 | 9,13E+03 | 1,58E+04 |
| 1 | 3,78E+03 | 2,26E+04 | 2,17E+04 | 3,84E+04 | 6,39E+04 |
| 10 | 4,52E+03 | 8,83E+04 | 8,43E+04 | 1,36E+05 | 1,91E+05 |
| 100 | 4.51E+04 | 3,58E+05 | 4,23E+05 | 4,95E+05 | 5,46E+05 |
| 500 | 3.62E+05 | 7,06E+05 | 6,90E+05 | 1,07E+06 | 1,28E+06 |
| 1000 | 5.51E+05 | 8,82E+05 | 1,00E+06 | 1,59E+06 | 1,85E+06 |

daraus resultierende dose-response-Charakteristika:

| Signalquotienten (Dynamiksteigerung) auf 1X lin normiert | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 1,00 | 1,56 | 1,19 | 2,70 | 4,6 |
| 1 | 1,00 | 5,98 | 5,74 | 10,16 | 16,90 |
| 10 | 1,00 | 19.54 | 18,65 | 30,09 | 42,2 |
| 100 | 1,00 | 7,94 | 9.38 | 10,98 | 12,11 |
| 500 | 1,00 | 1,95 | 1,91 | 2,96 | 3,54 |
| 1000 | 1,00 | 1,60 | 1,81 | 2,89 | 3,36 |

| Signalzuwächse absolut, bezogen auf 1X lin | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | | 1,89E+03 | 6,40E+02 | 5,75E+03 | 1,24E+04 |
| 1 | | 1,88E+04 | 1,79E+04 | 3,46E+04 | 6.01E+04 |
| 10 | | 8,38E+04 | 7,98E+04 | 1,31E+05 | 1,86E+05 |
| 100 | | 3,13E+05 | 3,78E+05 | 4,50E+05 | 5.01E+05 |
| 500 | | 3.44E+05 | 3,28E+05 | 7,08E+05 | 9.18E+05 |
| 1000 | | 3,31E+05 | 4,49E+05 | 1,04E+06 | 1,30E+06 |

Die unspezifische Bindung steigt mit wachsendem DNP-Einbau moderat an, vor allem aber ist ein enormer Anstieg der spezifischen Bindung als Funktion der DNP-Markierungsstärke zu verzeichnen, insbesondere im unteren Konzentrationsbereich. Je hoher der DNP-Einbau, desto besser die Steilheit der Kurve. In anderen Worten die erfindungsgemäße Signalamplifikation erlaubt eine wesentlich bessere Differenzierung kleiner Analytkonzentrationen von "Null", wie sowohl die Signalquotienten als auch die absoluten Signalzuwachse relativ zu 1X lin belegen.
Dies wird unterstrichen durch Berechnungen der korrespondierenden analytischen Sensitivität via linearer Regression zwischen 0 - 10 pmol/l auf Basis der zweifachen Standardabweichung uber dem Mittelwert einer Quadruplikatbestimmung des Nullwertes.

| | Steilheit 0-10 pM [RLU] | Nullwertsprazision [% VK] | UNG [pmol/l] |
|---|---|---|---|
| 1X lin | 1140 | 11.23 | 6.65 |
| 2X lin | 83030 | 16.32 | 0.21 |
| 2X' lin | 80280 | 40.01 | 0 40 |
| 6X bNA | 126870 | 2 83 | 0.04 |
| 8X bNA | 175200 | 9 30 | 0.167 |

Die außerordentlich gute UNG (= untere Nachweisgrenze) bei 6X bNA ist bedingt durch die in diesem Fall extrem gute Nullwertspräzision. Insgesamt gilt UNG = f⁻¹. (X DNP).

Aus Tabelle 1 ist daruber hinaus aber auch zu entnehmen, daß die erhöhte spezifische Signalgenerierung als Funktion der DNP-Markierungsstärke über die gesamte Eichkurve hinweg gelingt .

Das erfindungsgemaße Konzept wird zusatzlich bestätigt durch die Tatsache, daß sich die spezifische Bindung mit 2X lin- und 2X' lin-Sonden nicht signifikant unterscheiden, d.h. 2 Hapten-Markierungen führen via Reaktion mit MAK<DNP>Fab-Aequorin zu einer fast identischen Signalsteigerung gegenüber 1X lin, egal, ob die beiden Markierungen 9 nt oder 19 nt voneinander getrennt positioniert sind. In anderen Worten: mit flexibler Spacerung der Hapten-Markierungen (hier 3-Aminopropyl-triethylenglycol-glyceryl-Spacer im DNP-TEG) und sterisch wenig anspruchsvollem Anti-Hapten-Konjugat (hier <DNP>Fab-Aequorin[1.1], M, ca 70 kD, uber 14 Atom-Linker flexibel verknupft) lassen sich gegenüber dem Stand der Technik sehr eng gesetzte Markierungspunkte funktionell umsetzen.

### Beispiel 6B

Versuhsdurchführung wie in Beispiel 6A, jedoch mit einem um Faktor 10 auf 10000 pmol/l erweiterten Meßbereich und 0 15% RSA-c im Konjugatverdünnungspuffer

Wiederum bestätigen die Resultate (siehe Tabelle 2) das erfindungsgemäße Konzept in doppelter Weise
- 2X lin- und 2X' lin-Sonden sind bezüglich Generierung von spezifische Bindung ununterscheidbar, d h. hohe *Markierungsdichte* ist voll umsetzbar
- spezifische Bindung ist durchgehend streng eine Funktion der Anzahl der eingeführten DNP-Markierungen, d h *Pluralnat der Markierungspunkie* ist voll umsetzbar.

**Tabelle 2**

| DNA-Hybridisierungsimmunoassay mit DNP-markierten Indikatorsonden | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 4,38E+03 | 6,47E+03 | 4,76E+03 | 7,82E+03 | 1,45E+04 |
| 1 | 4,07E+03 | 1 12E+04 | 1,26E+04 | 1,89E+04 | 3,02E+04 |
| 10 | 5,07E+03 | 7,05E+04 | 7,49E+04 | 9,68E+04 | 1,53E+05 |
| 100 | 3,98E+04 | 3,57E+05 | 3,63E+05 | 4,78E+05 | 4,87E+05 |
| 1000 | 4,78E+05 | 8,36E+05 | 8,72E+05 | 1,34E+06 | 1,61E+06 |
| 10000 | 2,05E+06 | 3,26E+06 | 3,28E+06 | 4,27E+06 | 5,02E+06 |

daraus resultierende dose-response-Charakteristika

| Signalquotienten (Dynamiksteigerung) auf 1X lin normiert | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 1 | 1,48 | 1,09 | 1,79 | 3,31 |
| 1 | 1 | 2,75 | 3,10 | 4,64 | 7,42 |
| 10 | 1 | 13,91 | 14,77 | 19,09 | 30,18 |
| 100 | 1 | 8,97 | 9,12 | 12,01 | 12,24 |
| 1000 | 1 | 1,75 | 1,82 | 2,80 | 3,37 |
| 10000 | 1 | 1,59 | 1,60 | 2,08 | 2,45 |

| Signalzuwächse absolut, bezogen auf 1X lin | | | | | |
|---|---|---|---|---|---|
| Analyt = 20nt-Oligo-5'-Biotin [pmol/l] | gebundene Signale [RLU] | | | | |
| | IX lin | 2X lin | 2X' lin | 6X bNA | 8X bNA |
| 0 | | 2.09E+03 | 3,80E+02 | 3,44E+03 | 1,01E+04 |
| 1 | | 7,13E+03 | 8,53E+03 | 1,48E+04 | 2,61E+04 |
| 10 | | 6,54E+04 | 6,98E+04 | 9,17E+04 | 1,48E+05 |
| 100 | | 3,17E+05 | 3,23E+05 | 4,38E+05 | 4,47E+05 |
| 1000 | | 3,58E+05 | 3.94E+05 | 8,62E+05 | 1,13E+06 |
| 10000 | | 1.21E+06 | 1,23E+06 | 2,22E+06 | 2,97E+06 |

### Beispiel 6C

Der Meßbereich betrug hier wieder 0 - 1000 pmol/l, zur Reduktion von unspezifischer .Bindung wurde dem Testpuffer 200 mM Na₂-Tartrat x 2H₂O zugesetzt (=> salzreiches Puffermilieu) und die DNP-Sondenkonzentration auf jeweils 5 nmol/l herabgesetzt. Tatsächlich wurde die unspezifische Bindung mehr als halbiert, in bezug auf die spezifische Bindung wurden die zuvor beschriebenen Ergebmsse und damit das erfindungsgemäße Konzept aufs Neue bestatigt; siehe dazu Tabelle 3

**Tabelle 3.**

| DNA-Hybridisierungsimmunoassay mit DNP-markierten Indikatorsonden | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 2.72E+03 | 2,54E+03 | 4,02E+03 | 4,58E+03 | 6,86E+03 |
| 1 | 2,65E+03 | 7,54E+03 | 6,88E+03 | 1,04E+04 | 1,54E+04 |
| 10 | 2,72E+03 | 4,04E+04 | 3,66E+04 | 6,25E+04 | 9,66E+04 |
| 50 | 5,79E+03 | 1,56E+05 | 1,72E+05 | 2,50E+05 | 3,17E+05 |
| 100 | 1,49E+04 | 2,37E+05 | 2,59E+05 | 3,74E+05 | 3,93E+05 |
| 1000 | 3,12E+05 | 5,85E+05 | 6,25E+05 | 8,35E+05 | 9,29E+05 |

daraus resultierende dose-response-Charakteristika.

| Signalquotienten (Dynamiksteigerung), auf 1X lin normiert | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 1 | 0,93 | 1.48 | 1,68 | 2,52 |
| 1 | 1 | 2,85 | 2,60 | 3,92 | 5,81 |
| 10 | 1 | 14,85 | 13,46 | 22,98 | 35,50 |
| 50 | 1 | 26,94 | 29.71 | 43,18 | 54,66 |
| 100 | 1 | 15,91 | 17,38 | 25,10 | 26,34 |
| 1000 | 1 | 1,88 | 2,00 | 2,68 | 2,98 |

| Signalzuwächse absolut, bezogen auf 1X lin | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | | -1,80E+02 | 1,30E+03 | 1,86E+03 | 4,14E+03 |
| 1 | | 4,89E+03 | 4,23E+03 | 7,75E+03 | 1,28E+04 |
| 10 | | 3,77E+04 | 3,39E+04 | 5,98E+04 | 9,38E+04 |
| 50 | | 1,50E+05 | 1,66E+05 | 2,44E+05 | 3,11E+05 |
| 100 | | 2,22E+05 | 2,44E+05 | 3,59E+05 | 3,78E+05 |
| 1000 | | 2,73E+05 | 3,13E+05 | 5,23E+05 | 6,17E+05 |

### Beispiel 6D

Versuchsdurchführung analog Beispiel 6C, jedoch mit 4d/+2-8°C gelagerten Reagenzien und jeweils 10 nM Indikatorsonde (verdunnt aus der ebenfalls 4d/+2-8°C gelagerten 100 nM Vorverdünnung, aus der in Beispiel 6C die 5 nM Reagenzlösung hergestellt worden war).

Die Resultate sind in Tabelle 4 zusammengestellt .

**Tabelle 4**

| DNA-Hybridisierungsimmunoassay mit DNP-markierten Indikatorsonden | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 1,85E+03 | 2,36E+03 | 1,23E+03 | 1,33E+03 | 4,94E+03 |
| 1 | 2,17E+03 | 4,27E+03 | 3,26E+03 | 6,00E+03 | 7,96E+03 |
| 10 | 2,38E+03 | 1,16E+04 | 1,21E+04 | 2,00E+04 | 2,75E+04 |
| 50 | 2,94E+03 | 4,49E+04 | 4,81E+04 | 9,49E+04 | 1,28E+05 |
| 100 | 3,41E+03 | 7.83E+04 | 8,90E+04 | 1,69E+05 | 2,07E+05 |
| 1000 | 1,00E+05 | 3,63E+05 | 3,33E+05 | 5,24E+05 | 5,42E+05 |

daraus resultierende dose-response-Charakteristika.

| Signalquotienten (Dynamiksteigerung), auf 1X lin normiert | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | 1 | 1,28 | 0,66 | 0,72 | 2,67 |
| 1 | 1 | 1,97 | 1,50 | 2,76 | 3,67 |
| 10 | 1 | 4,87 | 5,08 | 8,40 | 11,55 |
| 50 | 1 | 15,27 | 16,36 | 32,28 | 43,54 |
| 100 | 1 | 22,96 | 26,10 | 49,56 | 60,70 |
| 1000 | 1 | 3,63 | 3,33 | 5,24 | 5,42 |

| Signalzuwächse absolut, bezogen auf 1X lin | | | | | |
|---|---|---|---|---|---|
| Analyt = 20nt-Oligo-5'-Biotin [pmol/l] | gebundene Signale [RLU] | | | | |
| | 1X lin | 2X lin | 2X' lin | 6X bNA | 8X bNA |
| 0 | | 5,10E+02 | -6,20E+02 | -5,20E+02 | 3,09E+03 |
| 1 | | 2,10E+03 | 1,09E+03 | 3,83E+03 | 5,79E+03 |
| 10 | | 9,22E+03 | 9,72E+03 | 1,76E+04 | 2,51E+04 |
| 100 | | 4,20E+04 | 4,52E+04 | 9,20E+04 | 1,25E+05 |
| 500 | | 7,49E+04 | 8,56E+04 | 1,66E+05 | 2,04E+05 |
| 1000 | | 2,63E+05 | 2,33E+05 | 4.24E+05 | 4,42E+05 |

Fazit: Vielfachmarkierung via Hapten und hohe Markierungsdichte führen keinesfalls zu einer verminderten Stabilität in Lösung, eher im Gegenteil. In anderen Worten; das erfindungsgemäße Konzept einer Signalamplifikation bedingt mit Hinblick auf Stabilität keine Einschränkung in puncto Praktikabilität

| maximale spezifische Bindung (1000 pmol/l Analyt) | |
|---|---|
| 1X lin | 32% recovery relativ zu Beispiel 6C (= frisch) |
| 2X lin | 62% |
| 2X' lin | 53% |
| 6X bNA | 62% |
| 8X bNA | 60% |

Auch hier, mit belasteten Reagenzien wird der Vorteil der erfindungsgemäßen Signalamplifikation nicht nur anhand von Signalquotienten und Signalzuwachsen deutlich (beachte insbesondere auch die Steigerungen beim Ubergang 6X bNA zu 8X bNA), sondern wird auch unterstrichen durch UNG-Berechnungen (analog Beispiel 6A), in die die Nullwertspräzision gewichtet uber die Kurvensteilheit eingeht:

| | analytische Sensitivität = UNG [pmol/l] |
|---|---|
| 1X lin | 21.80 |
| 2X lin | 0.89 |
| 8X bNA | 0.39 |

### Abkürzungen

- DNP: Dinitrophenyl
- HBV: Hepatitis B Virus
- SA: Streptavidin
- RSA: Rinderserumalbumin
- RT: Raumtemperatur
- MAK<X>: Monoklonaler Antikörper gegen X
- DIG: Digoxigenin
- RLU: Relative Light Units
- VK: Variationskoeffizient

### SEQUENZPROTOKOLL

(1) ALLGEMEINE ANGABEN
   (i) ANMELDER.
      (A) NAME. Boehringer Mannheim GmbH
      (B) STRASSE Sandhoferstr 116
      (C) ORT Mannheim
      (E) LAND DE
      (F) POSTLEITZAHL 68298
      (G) TELEFON 06217594348
      (H) TELEFAX 06217594457
   (ii) BEZEICHNUNG DER ERFINDUNG Verfahren zum Nachweis eines Analyten
   (iii) ANZAHL DER SEQUENZEN 7
   (iv) COMPUTER-LESBARE FASSUNG
      (A) DATENTRAGER Floppy disk
      (B) COMPUTER: IBM PC comparable
      (C) BETRIEBSSYSTEM PC-DOS/MS-DOS
      (D) SOFTWARE Patentin Release #1.0. Version #1 30 (EPA)
(2) ANGABEN ZU SEQ ID NO 1
   (i) SEQUENZKENNZEICHEN
      (A) LÄNGE. 27 Basenpaare
      (B) ART: Nucleotid
      (C) STRANGFORM. Einzelstrang
      (D) TOPOLOGIE: lincar
   (ii) ART DES MOLEKULS Sonstage Nucleinsäure
      (A) BESCHREIBUNG /desc = "Oligodesoxynbonukleotid"
   (iii) HYPOTHETISCH NEIN
   (ix) MERKMAL.
      (A) NAME/SCHLUSSEL misc_feature
      (B) LAGE:1.27
      (D) SONSTIGE ANGABEN/note= "Das Nukleotid in Position I enthaelt Biotin gebunden Y bedeutet 3-Amino-1,2-propandiol, mit 6-Aminohexansaeure verlaengert und mit Digoxigenin-N-hydroxysuccinimidester markiert."
   (xi) SEQUENZBESCHREIBUNG SEQ ID NO: 1
(2) ANGABEN ZU SEQ ID NO 2
   (i) SEQUENZKENNZEICHEN
      (A) LANGE. 31 Basenpaare
      (B) ART. Nucleotid
      (C) STRANGFORM Einzelstrang
      (D) TOPOLOGIE: lincar
   (u) ART DES MOLEKULS Sonstige Nucleinsaure
      (A) BESCHREIBUNG /desc = "Oligodesoxynbonukleotid"
   (ix) MERKMAL
      (A) NAME/SCHLUSSEL misc feature
      (B) LAGE 1
      (D) SONSTIGE ANGABEN /note= "Y bedeutet DNP mit Hilfe von DNP-TEG gebunden"
   (xi) SEQUENZBESCHREIBUNG SEQ ID NO 2.
(2) ANGABEN ZU SEQ ID NO 3
   (i) SEQUENZKENNZEICHEN
      (A) LÄNGE: 20 Basenpaare
      (B) ART: Nucleotid
      (C) STRANGFORM Einzelstrang
      (D) TOPOLOGIE lincar
   (u) ART DES MOLEKULS Sonstage Nucleinsaure
      (A) BESCHREIBUNG /desc = "Oligodesoxynbonukleotid"
   (ix) MERKMAL
      (A) NAME/SCHLUSSEL misc_feature
      (B) LAGE: 1
      (D) SONSTIGE ANGABEN:/note= "am 5'-Ende ist Biotin ueber Aminolinker AMII (Bochnnger Mannheim) gebunden"
   (xi) SEQUENZBESCHREIBUNG. SEQ ID NO 3
   AGACCACCAA ATGCCCCTAT 20
(2) ANGABEN ZU SEQ ID NO 4:
   (i) SEQUENZKENNZEICHEN
      (A) LÄNGE 41 Basenpaare
      (B) ART Nucleotid
      (C) STRANGFORM: Einzelstrang
      (D) TOPOLOGIE linear
   (ii) ART DES MOLEKÜLS: Sonstige Nucleinsaure
      (A) BESCHREIBUNG /desc = "Oligodesoxynbonukleotid"
   (ix) MERKMAL
      (A) NAME/SCHLUSSEL misc_feature
      (B) LAGE.I
      (D) SONSTIGE ANGABEN./note= "Am 5'-Ende ist DNP mit Hlife von DNP-TEG gebunden"
   (ix) MERKMAL
      (A) NAME/SCHLUSSEL misc_feature
      (B) LAGE. 11
      (D) SONSTIGE ANGABEN /note= "Y bedeutet DNP ueber DNP-TEG gebunden"
   (xi) SEQUENZBESCHREIBUNG SEQ ID NO 4
(2) ANGABEN ZU SEQ ID NO 5
   (i) SEQUENZKENNZEICHEN
      (A) LANGE. 51 Basenpaare
      (B) ART: Nucleotid
      (C) STRANGFORM Einzelstrang
      (D) TOPOLOGIE. linear
   (ii) ART DES MOLEKULS Sonstige Nucleinsaure
      (A) BESCHREIBUNG /desc = "Oligodesoxynbonukleotid"
   (ix) MERKMAL
      (A) NAME/SCHLUSSEL misc_feature
      (B) LAGE. 1
      (D) SONSTIGE ANGABEN/note= "Y bedeutet DNP ueber DNP-TEG gebunden"
   (ix) MERKMAL
      (A) NAME/SCHLUSSEL. misc_feature
      (B)LAGE:21
      (D) SONSTIGE ANGABEN/note="Y bedeutet DNP ueber DNP-TEG eingebaut"
   (xi) SEQUENZBESCHREIBUNG SEQ ID NO 5
(2) ANGABEN ZU SEQ ID NO 6
   (i) SEQUENZKENNZEICHEN
      (A) LANGE 65 Basenpaare
      (B) ART: Nucleotid
      (C) STRANGFORM Einzelstrang
      (D) TOPOLOGIE linear
   (u) ART DES MOLEKULS Sonstage Nucleinsäure
      (A) BESCHREIBUNG /desc = "Oligodesoxybonukleotid"
   (ix) MERKMAL
      (A) NAME/SCHLUSSEL misc_feature
      (B) LAGE. 1
      (D) SONSTIGE ANGABEN /note= "Y bedeutet DNP ueber DNP-TEG gebunden"
   (ix) MERKMAL
      (A) NAME/SCHLUSSEL misc_feature
      (B) LAGE. 11
      (D) SONSTIGE ANGABEN/note= "Y bedeutet DNP ueber DNP-TEG eingebaut"
   (ix) MERKMAL
      (A) NAME/SCHLUSSEL misc_feature
      (B) LAGE.24
      (D) SONSTIGE ANGABEN /note= "Y bedeutet DNP ueber DNP-TEG eingebaut"
   (ix) MERKMAL
      (A) NAME/SCHLUSSEL misc_feature
      (B) LAGE.29
      (D) SONSTIGE ANGABEN /note= "Y bedeutet hier AM W Maleinimido oder AM III-Maleinimido-S-TTTTTTTTTYTTTTTTTTYT-3'. wobei Y DNP eingebaut ueber DNP-TEG bedeutet"
   (ix) MERKMAL
      (A) NAME/SCHLUSSEL misc_feature
      (B) LAGE.34
      (D) SONSTIGE ANGABEN/note= "Y bedeutet DNP ueber DNP-TEG eingebaut"
   (ix) MERKMAL
      (A) NAME/SCHLUSSEL misc_feature
      (B) LAGE.39
      (D) SONSTIGE ANGABEN /note= "Y ist definiert wie in Position 29"
   (xi) SEQUENZBESCHREIBUNG SEQ ID NO 6
(2) ANGABEN ZU SEQ ID NO 7
   (i) SEQUENZKENNZEICHEN
      (A) LANGE 61 Basenpaare
      (B) ART Nucleotid
      (C) STRANGFORM Einzelstrang
      (D) TOPOLOGIE linear
   (ii) ART DES MOLEKULS Sonstige Nucleinsäure
      (A) BESCHREIBUNG /desc = "Oligodesoxynbonukleotid"
   (ix) MERKMAL
      (A) NAME/SCHLUSSEL misc_feature
      (B) LAGE: I
      (D) SONSTIGE ANGABEN /note= "Y bedeutet DNP ueber DNP-TEG gebunden"
   (ix) MERKMAL
      (A) NAME/SCHLUSSEL misc_feature
      (B) LAGE: 11
      (D) SONSTIGE ANGABEN./note= "Y bedeutet DNP ueber DNP-TEG eingebaut"
   (ix) MERKMAL
      (A) NAME/SCHLUSSEL misc_feature
      (B) LAGE 21
      (D) SONSTIGE ANGABEN /note= "Y bedeutet DNP ueber DNP-TEG eingebaut"
   (ix) MERKMAL.
      (A) NAME/SCHLUSSEL. misc_feature
      (B) LAGE.26
      (D) SONSTIGE ANGABEN /note= "v bedeutet AM
         III-Maleinimido oder AM
         III-Maleinimido-S-TTTTTTTTTTTTTTTTTTTYT-3'. wobei Y DNP ueber DNP-TEG eingebaut bedeutet"
   (ix) MERKMAL.
      (A) NAME/SCHLUSSEL misc_feature
      (B) LAGE 31
      (D) SONSTIGE ANGABEN /note= "Y bedeutet DNP ueber DNP-TEG eingebaut"
   (ix) MERKMAL
      (A) NAME/SCHLUSSEL misc_feature
      (B) LAGE 36
      (D) SONSTIGE ANGABEN /note= "Y hat die Bedeutung wie in Postion 26"
   (xi) SEQUENZBESCHREIBUNG SEQ ID NO 7

### Bezugszeichenliste

1. Boden oder Wand einer Mikrotiterplatte
2 Aktive Festphase, bestehend aus einer (Strept)Avidin-Beschichtung der Testträgerwand, plus einer darauf immobilisierharen biotinylierten Festphasensonde, deren Sequenz komplementär zu einer Teilsequenz der Fangsonde (3) ist
3 Fangsonde mit einer zu der nachzuweisenden Nukleinsäure komplementären Nukleotid-Teilsequenz und einer zu der Festphasensonde komplementäre Teilsequenz
4. Nachzuwersende Nukleinsäure
5. Oligonukleotid enthaltend einen zu einem Teil der nachzuweisenden Nukleinsäure komplementären Teil und einem universellen, zu einem Teil der nukleobasenhaltigen Sonde (6) komplementären Teil
6 Erfindungsgemäße Sonde mit kammartig von einem Grundgerüst abstehenden Seitenarmen
7 An die Primarsequenz oder die Sekundarsequenzen gebundene nicht-nukleosidische markierungsvermittelnde Gruppe (z B Digoxigenin)
8 Konjugat aus einem Bindepartner der nicht-nukleosidischen markierungsvermittelnden Gruppe und einem kalziumabhängigen Photoprotein (z. B. Antidigoxigenin-Aequorin)

## Patentansprüche

1. Sonde zum Nachweis eines Analyten, **dadurch gekennzeichnet, dass** sie aufgebaut ist aus
- einem analytspezifischen Teil, mit dem die Sonde den Analyten direkt oder indirekt binden kann, und
- einem Teil, der Nukleobasen an einem Backbone enthält,
der über Verzweigungsmonomere mehrfach in sich verzweigt ist und eine zentrale Primärsequenz mit mehreren Verzweigungspunkten und an die Verzweigungspunkte geknüpfte periphere Sekundärsequenzen enthält,
welcher nicht-nukleosidische, markierungsvermittelnde Gruppen enthält, wobei der Abstand zwischen den Mitten zweier aufeinanderfolgender mit einer markierungsvermittelnden Gruppe markierter Mononukleoside der Sonde kleiner ist als 18 Nukleotide.

2. Sonde gemäß Anspruch 1, **dadurch gekennzeichnet, dass** der Abstand zwischen Verzweigungspunkten kleiner ist als 11, bevorzugt weniger als 7 Nukleotide.

3. Sonde gemäß einem der Ansprüche 1 bis 2, **dadurch gekennzeichnet, dass** die markierungsvermittelnden Gruppen und der Sonden-Backbone über einen Spacer von ≥ 4 Atomen verknüpft sind.

4. Sonde gemäß einem der Ansprüche 1 bis 3, **dadurch gekennzeichnet, daß** die markierungsvermittelnden Gruppen in die Primär- und Sekuridärsequenz eingebaut sind.

5. Verfahren zum Nachweis eines Analyten in einer Probe durch Kontaktieren der Probe mit einer nukleobasenhaltigen Sonde gemäß einem der Ansprüche 1 bis 4 unter Bedingungen, bei denen der Analyt mittelbar oder unmittelbar an die Sonde bindet, und Nachweis des Bindungsprodukts.

6. Verfahren gemäß Anspruch 5, **dadurch gekennzeichnet, dass** das Bindeprodukt über ein Konjugat nachgewiesen wird, welches eine molare Masse von ≤ 100 kDa hat und welches aus einer zur markierungsvermittelnden Gruppe affinen Gruppe und einer damit über einen Linker mit einer Länge von ≥ 4 Atomen verknüpften signalgebenden Komponente besteht.

7. Verfahren gemäß Anspruch 6, **dadurch gekennzeichnet, dass** die markierungsvermittelnde Gruppe ein Hapten ist.

8. Verfahren gemäß einem der Ansprüche 5 bis 7, **dadurch gekennzeichnet, dass** die signalgebende Komponente ein natives oder rekombinant hergestelltes Calciumaktivierbares Photoprotein ist.

9. Testkit zur Durchführung eines Verfahrens gemäß einem der Ansprüche 5 bis 8, **dadurch gekennzeichnet, dass** es eine Sonde gemäß einem der Ansprüche 1 bis 4 sowie alle zur Erzeugung eines Meßsignals erforderlichen Reagenzien beinhaltet.

10. Verwendung einer Sonde gemäß einem der Ansprüche 1 bis 4 zum Nachweis eines Analyten in einer Probe.

## Claims

1. Probe for detecting an analyte, **characterized in that** it is composed of
- an analyte-specific part by means of which the probe can directly or indirectly bind the analyte and
- a part which contains nucleobases on a backbone,
which is multiply internally branched by branching monomers and contains a central primary sequence with several branching points and peripheral secondary sequences linked to the branching points,
which contains non-nucleosidic, label-mediating groups where the distance between the midpoints of two consecutive mononucleosides of the probe that are labelled with a label-mediating group is less than 18 nucleotides.

2. Probe according to claim 1, **characterized in that** the distance between branching points is less than 11, preferably less than 7 nucleotides.

3. Probe according to one of the claims 1 to 2, **characterized in that** the label-mediating groups and the probe backbone are connected by a spacer of ≥ 4 atoms.

4. Probe according to one of the claims 1 to 3, **characterized in that** the label-mediating groups are incorporated into the primary and secondary sequence.

5. Method for detecting an analyte in a sample by contacting the sample with a probe which contains nucleobases according to one of the claims 1 to 4 under conditions in which the analyte binds directly or indirectly to the probe, and detecting the binding product.

6. Method according to claim 5, **characterized in that** the binding product is detected by means of a conjugate which has a molar mass of ≤ 100 kDa and which consists of a group that has affinity to the label-mediating group and a signal-generating component which is linked thereto by means of a linker of ≥ 4 atoms in length.

7. Method according to claim 6, **characterized in that** the label-mediating group is a hapten.

8. Method according to one of the claims 5 to 7, **characterized in that** the signal-generating component is a native or recombinantly produced calcium-activatable photoprotein.

9. Test kit for carrying out a method according to one of the claims 5 to 8, **characterized in that** it contains a probe according to one of the claims 1 to 4 and all reagents required to generate a measuring signal.

10. Use of a probe according to one of the claims 1 to 4 to detect an analyte in a sample.

## Revendications

1. Sonde pour la mise en évidence d'un analyte, **caractérisée en ce qu'**elle est élaborée à partir
- d'une partie spécifique d'un analyte, avec laquelle la sonde peut lier l'analyte directement ou indirectement et
- d'une partie qui contient des bases nucléiques sur un squelette
qui est ramifiée en soi plusieurs fois via des monomères de ramification et qui contient une séquence primaire centrale avec plusieurs points de ramification et des séquences secondaires périphériques liées aux points de ramification,
qui contient des groupes non nucléosidiques, qui interviennent dans le marquage, la distance entre les centres de deux mononucléosides successifs marqués avec un groupe qui intervient dans le marquage de la sonde étant inférieure à 18 nucléotides.

2. Sonde selon la revendication 1, **caractérisée en ce que** la distance entre les points de ramification est inférieure à 11, de préférence inférieure à 7 nucléotides.

3. Sonde selon l'une quelconque des revendications 1 à 2, **caractérisée en ce que** les groupes qui interviennent dans le marquage et le squelette de la sonde sont liés via un écarteur présentant ≥ 4 atomes.

4. Sonde selon l'une quelconque des revendications 1 à 3, **caractérisée en ce que** les groupes qui interviennent dans le marquage sont incorporés dans la séquence primaire et secondaire.

5. Procédé pour la mise en évidence d'un analyte dans un échantillon par la mise en contact de l'échantillon avec une sonde contenant des bases nucléiques selon l'une quelconque des revendications 1 à 4 dans des conditions dans lesquelles l'analyte se lie directement ou indirectement à la sonde et la mise en évidence du produit de liaison.

6. Procédé selon la revendication 5, **caractérisé en ce que** le produit de liaison est mis en évidence via un produit conjugué qui présente une masse molaire ≤100 kDa et qui est constitué par un groupe à affiner en groupe qui intervient dans le marquage et un composant donnant le signal lié à celui-ci via un écarteur présentant une longueur de ≥4 atomes.

7. Procédé selon la revendication 6, **caractérisé en ce que** le groupe qui intervient dans le marquage est un haptène.

8. Procédé selon l'une quelconque des revendications 5 à 7, **caractérisé en ce que** le composant donnant le signal est une photoprotéine native ou une photoprotéine activable au calcium produite par recombinaison.

9. Kit de test pour réaliser un procédé selon l'une quelconque des revendications 5 à 8, **caractérisé en ce qu'**il contient une sonde selon l'une quelconque des revendications 1 à 4 ainsi que tous les réactifs nécessaires pour obtenir un signal de mesure.

10. Utilisation d'une sonde selon l'une quelconque des revendications 1 à 4 pour la mise en évidence d'un analyte dans un échantillon.
